(19) Europäisches Patentamt
European Patent Office
Office européen des brevets

(11) EP 1 743 642 A1

(12) EUROPEAN PATENT APPLICATION

(43) Date of publication:
17.01.2007 Bulletin 2007/03

(51) Int Cl.:
*A61K 31/4155* (2006.01) *A61K 31/496* (2006.01)
*A61K 31/454* (2006.01)

(21) Application number: 05384024.5

(22) Date of filing: 15.07.2005

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK YU

(71) Applicant: LABORATORIOS DEL DR. ESTEVE, S.A.
08041 Barcelona (ES)

(72) Inventors:
- Buschmann, Helmut Heinrich
  08960 Sant Just Desvern (Barcelona) (ES)
- Torrens Jover, Antonio
  08221 Terrasa (Barcelona) (ES)
- Yenes Minguez, Susana
  08750 Molins de Rei,
  Barcelona (ES)
- Mas Prio, José
  08191-Rubi,
  Barcelona (ES)
- Quintana Ruiz, Jordi Ramón
  08391-Tiana,
  Barcelona (ES)
- Dordal Zueras, Alberto
  08016 Barcelona (ES)
- Cubertes-Altisen, Maria Rosa
  San Cugat del Valles,
  Barcelona (ES)

(54) **Use of substituted pyrazoline compounds and their derivatives for the treatment of cannabinoid system-associated diseases**

(57) The present invention relates to the use of substituted pyrazoline compounds, their derivatives as well as their physiologically acceptable salts , in medicinal products for human therapeutics and/or animal research for the treatment of a variety of diseases associated the cannabinoid receptor system in humans and animals.

EP 1 743 642 A1

## Description

### Field of the invention

The present invention relates to the use of a substituted pyrazoline compound with the general formula (I),

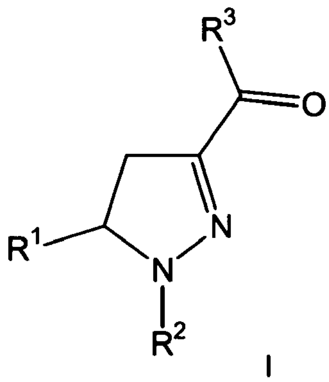

I

their derivatives as well as their physiologically acceptable salts; and a substituted pyrazoline compound with the general formula (II),

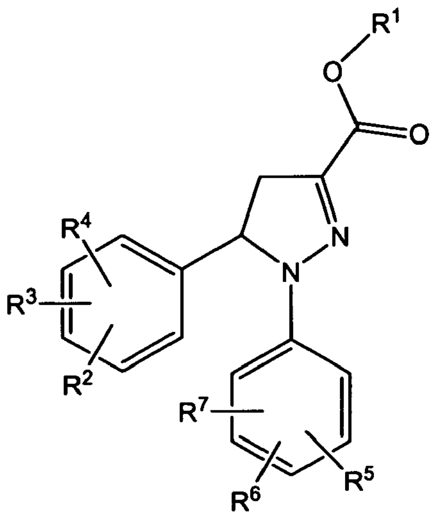

II

their derivatives as well as their physiologically acceptable salts in medicinal products for human therapeutics and/or animal research for the treatment of a variety of diseases associated the cannabinoid receptor system in humans and animals.

### Background of the invention

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of

physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Surprisingly it was found that substituted pyrazoline compounds have a beneficial effect on the treatment of a variety of diseases associated with the cannabinoid receptor system.

Therefore, the present invention relates to new forms of treatment for diseases associated with the cannabinoid receptor system with substituted pyrazoline compounds of the general formula (I),

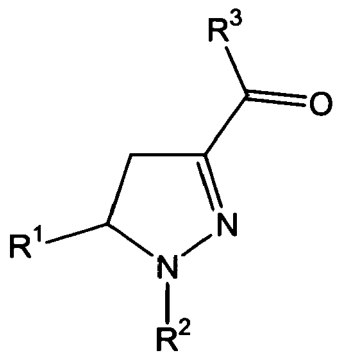

I

wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an $-NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an $-SO_2-R^6$-moiety, or an $-NR^7R^8$-moiety,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic

ring system and/or bonded via a linear or branched alkylene group,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferably the following provisos (disclaimers) apply for the pyrazoline compounds of general formula I given above:

that $R^4$ and $R^5$ do not both represent a hydrogen atom, and

that if one of the residues $R^4$ and $R^5$ represents a hydrogen atom or an alkyl group, which is optionally at least mono-substituted with an alkoxy group, an alkoxyalkoxy group, a halogen atom or a phenyl group, the other one of these residues $R^4$ and $R^5$ does not represent a pyrid-2-yl group, which is optionally mono-substituted in the 5-position, a pyrid-5-yl group, which is optionally mono-substituted in the 2-position, a pyrimid-5-yl group, which is optionally mono-substituted in the 2-position, a pyridaz-3-yl group, which is optionally mono-substituted in the 6-position, a pyrazin-5-yl group, which is optionally mono-substituted in the 2-position, a thien-2-yl group, which is optionally mono-substituted in the 5 position, a thien-2-yl group, which is optionally at least mono-substituted in the 4-position, a benzyl group, which is optionally mono-substituted in the 4-position of the ring, a phenethyl group, which is optionally mono-substituted in the 4-position of the ring, an optionally mono-, di- or tri-substituted phenyl group, a di-substituted phenyl group, wherein the two substituents together form an $-OCH_2O-$, $-OCH_2CH_2O-$ or $-CH_2CH_2O-$ chain, which is optionally substituted with one or more halogen atoms or one or two methyl groups, an —NH-phenyl-moiety, wherein the phenyl group may be mono-substituted in the 4-position, and

that if one of the residues $R^4$ and $R^5$ represents an alkynyl group, the other one of these residues $R^4$ and $R^5$ does not represent a phenyl group, which is optionally substituted in the 4-position, and

that if one of the residues $R^4$ and $R^5$ represents a hydrogen atom or a linear or branched, saturated or unsaturated, unsubstituted or substituted aliphatic radical, the other one of these residues $R^4$ and $R^5$ does not represent an unsubstituted or substituted thiazole group or an unsubstituted or substituted [1,3,4]thiadiazole group.

An additional aspect of the present invention relates to new ways of treatment for diseases associated with the cannabinoid receptor system with substituted pyrazoline compounds of the general formula (II),

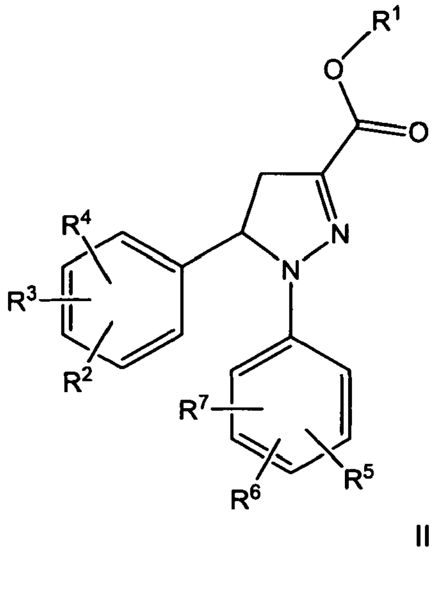

II

wherein

$R^1$ represents hydrogen or a linear or branched $C_{1-4}$-alkyl group,

$R^2$, $R^3$ and $R^4$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^8$, SH, $SR^8$, $SOR^8$, $SO_2R^8$, $NH_2$, $NHR^8$, $NR^8R^9$, -(C=O)-$NH_2$, -(C=O)-$NHR^8$ or -(C=O)-$NR^8R^9$ whereby $R^8$ and $R^9$ for each substituent independently represent linear or branched $C_{1-6}$ alkyl,

$R^5$ and $R^6$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10}$, SH, $SR^{10}$, $SOR^{10}$, $NH_2$, $NHR^{10}$, $NR^{10}R^{11}$, -(C=O)-$NH_2$, -(C=O)-$NHR^{10}$ and -(C=O)-$NR^{10}R^{11}$, whereby $R^{10}$ and optionally $R^{11}$ for each substituent independently represent linear or branched $C_{1-6}$ alkyl;

$R^7$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10}$, SH, $SR^{10}$, $SOR^{10}$, $NH_2$, $NHR^{10}$, $NR^{10}R^{11}$, -(C=O)-$NH_2$, -(C=O)-$NHR^{10}$ and -(C=O)-$NR^{10}R^{11}$, whereby $R^{10}$ and optionally $R^{11}$ for each substituent independently represent linear or branched $C_{1-6}$ alkyl;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Referring to compounds of general formula I,

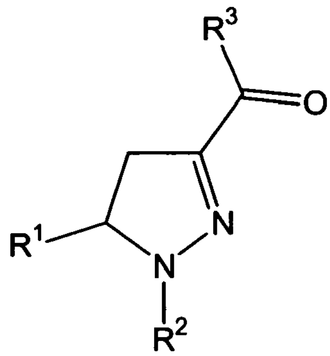

I

a mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more, e.g. 1, 2 or 3, heteroatoms as ring members, which may be identical or different and which can preferably be selected from the group consisting of N, O, S and P, more preferably be selected from the group consisting of N, O and S.

Preferably the polycyclic ring-system may comprise two rings that are condensed. The rings of the mono- or polycyclic ring-sytem are preferably 5- or 6-membered.

The term "condensed" according to the present invention means that a ring or ring-system is attached to another ring or ring-system, whereby the terms "annulated" or "annelated" are also used by those skilled in the art to designate this kind of attachment.

If one or more of the residues $R^3$-$R^8$ represents or comprises a saturated or unsaturated, optionally at least one heteroatom as ring member containing cycloaliphatic group, which is substituted by one or more, e.g. 1, 2, 3 or 4, substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, oxo, amino, carboxy, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, oxo, $CF_3$ and a phenyl group.

If one or more of the residues $R^3$-$R^8$ represents or comprises a cycloaliphatic group, which contains one or more heteroatoms as ring members, unless defined otherwise, each of these heteroatoms may preferably be selected from the group consisting of of N, O and S. Preferably a cycloaliphatic group may contain 1, 2 or 3 heteratoms independently selected from the group consisting of N, O and S as ring members.

Suitable saturated or unsaturated, optionally at least one heteroatom as ring member containing, optionally at least mono-substituted cycloaliphatic groups may preferably be selected from the group consisting of Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Pyrrolidinyl, Piperidinyl, Piperazinyl, homo-Piperazinyl and Morpholinyl.

If one or more of the residues $R^3$—$R^8$ comprises a mono- or polycyclic ring system, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-6}$-alkoxy, branched or unbranched $C_{1-6}$-alkyl, branched or unbranched $C_{1-4}$-perfluoroalkoxy, branched or unbranched $C_{1-4}$-perfluoroalkyl, amino, carboxy, oxo, amido, cyano, nitro, -$SO_2NH_2$, -CO-$C_{1-4}$-alkyl, -SO-$C_{1-4}$-alkyl, -$SO_2$-$C_{1-4}$-alkyl, -NH-$SO_2$-$C_{1-4}$-alkyl , wherein the $C_{1-4}$-alkyl may in each case be branched or unbranched, and a phenyl group, more preferably be selected from the group consisting of hydroxy, F, Cl, Br, methyl, ethyl, methoxy, ethoxy, $CF_3$, oxo and a phenyl group.

If one or more of the residues $R^1$-$R^8$ represents or comprises an aryl group, including a phenyl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a nitro group, a carboxy group, a -CO-O-$C_{1-6}$-alkyl group, a -CO-$NR^AR^B$-moiety, a -CO-NH-$NR^CR^D$-moiety, an -SH, an -S-$C_{1-6}$-alkyl group, an -SO-$C_{1-6}$-alkyl group, an -$SO_2$-$C_{1-6}$-alkyl group, a -$C_{1-6}$-alkyleneS-$C_{1-6}$-alkyl group, a -$C_{1-6}$-alkylene-SO-$C_{1-6}$-alkyl group, a -$C_{1-6}$-alkylene-$SO_2C_{1-6}$-alkyl group, an -$NH_2$-moiety, an NHR'-moiety or an NR'R"-moiety, wherein R' and R" independently represent a linear or branched $C_{1-6}$-alkyl group, a $C_{1-6}$-alkyl group substituted by one or more hydroxy groups and a -$C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a- CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a -CO-O- $C_{1-9}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a -CO-$NH_2$ group and/or which may contain at least one further heteroatom selected from the group consisting of nitrogen, oxygen and sulphur as a ring member, and
wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member.

Preferred aryl groups, which may optionally be at least mono-substituted, are phenyl and naphthyl.

If one or more of the residues $R^3$-$R^8$ represents or comprises a heteroaryl group, which is substituted by one or more, e.g. 1, 2, 3, 4 or 5 substituents, unless defined otherwise, each of the substituents may be independently selected from the group consisting of a halogen atom (e.g. F, Cl, Br, I), a linear or branched $C_{1-6}$-alkyl group, a linear or branched $C_{1-6}$ alcoxy group, a formyl group, a hydroxy group, a trifluoromethyl group, a trifluoromethoxy group, a -CO-$C_{1-6}$-alkyl group, a cyano group, a carboxy group, a —CO-O-$C_{1-6}$-alkyl group, a —CO-$NR^AR^B$- moiety, a -CO-NH-$NR^CR^D$-moiety, an -S-$C_{1-6}$-alkyl group, an -SO-$C_{1-6}$-alkyl group, an -$SO_2$-$C_{1-6}$-alkyl group, a -$C_{1-6}$-alkylene-S-$C_{1-6}$-alkyl group, a -$C_{1-6}$-alkylene-SO-$C_{1-6}$-alkyl group, a -$C_{1-6}$-alkylene-$SO_2$-$C_{1-6}$-alkyl group, a $C_{1-6}$-alkyl group substituted by one or more hydroxy groups and a —$C_{1-6}$-alkylene-$NR^ER^F$ group,
whereby $R^A$, $R^B$, identical or different, represent hydrogen or a $C_{1-6}$-alkyl group, or $R^A$ and $R^B$ together with the bridging nitrogen atom form a saturated, mono- or bicyclic, 3-10 membered heterocyclic ring system, which may be at least mono-substituted by one or more, identical or different, $C_{1-6}$ alkyl groups and/or which may contain at least one further heteroatom selected from the group consisting of nitrogen, oxygen and sulphur as a ring member,
$R^C$, $R^D$, identical or different, represent a hydrogen atom, a $C_{1-6}$-alkyl group, a -CO-O-$C_{1-6}$-alkyl group, a $C_{3-8}$-cycloalkyl group, a $C_{1-6}$-alkylene-$C_{3-8}$-cycloalkyl group, $C_{1-6}$-alkylene-O-$C_{1-6}$-alkyl group or a $C_{1-6}$-alkyl group substituted with one or more hydroxy groups, or $R^C$, $R^D$ together with the bridging nitrogen atom form a saturated, mono- or bicyclic, 3-10 membered heterocyclic ring system, which may be at least mono-substituted by one or more substituents independently selected from the group consisting of $C_{1-6}$ alkyl group, a -CO-$C_{1-6}$-alkyl group, a -CO-O- $C_{1-6}$-alkyl group, a -CO-NH-$C_{1-6}$-alkyl group, a -CS-NH- $C_{1-6}$-alkyl group, an oxo group, a $C_{1-6}$-alkyl group substituted with one or more hydroxy groups, a $C_{1-6}$-alkylene-O-$C_{1-6}$-alkyl group and a -CO-$NH_2$ group and/or which may contain at least one further heteroatom selected from the group consisting of nitrogen, oxygen and sulphur as a ring member, and

wherein $R^E$, $R^F$, identical or different, represent hydrogen or a $C_{1-6}$-alkyl group, or $R^E$ and $R^F$ together with the bridging nitrogen atom form a saturated, mono- or bicyclic, 3-10 membered heterocyclic ring system, which may be at least mono-substituted by one or more, identical or different $C_{1-6}$ alkyl groups and/or which may contain at least one further heteroatom selected from the group consisting of nitrogen, oxygen and sulphur as a ring member,

The heteroatoms, which are present as ring members in the heteroaryl radical, may, unless defined otherwise, independently be selected from the group consisting of nitrogen, oxygen and sulphur. Preferably a heteroaryl radical may comprise 1, 2 or 3 heteroatoms independently selected from the group consisting of N, O and S as ring members.

Suitable heteroaryl groups, which may optionally be at least mono-substituted, may preferably be selected from the group consisting of thienyl, furyl, pyrrolyl, pyridinyl, imidazolyl, pyrimidinyl, pyrazinyl, indolyl, chinolinyl, isochinolinyl, benzo[1,2,5]-thiodiazolyl, benzo[b]thiophenyl, benzo[b]furanyl, imidazo[2,1-b]thiazolyl, triazolyl, and pyrazolyl, more preferably be selected from the group consisting of thienyl-, benzo[1,2,5]-thiodiazolyl, benzo[b]thiophenyl, imidazo [2,1-b]thiazolyl, triazolyl and pyrazolyl.

If one or more of the residues $R^4$-$R^8$ represents or comprises a linear or branched, saturated or unsaturated aliphatic group such as an alkyl group, which is substituted by one or more, e.g. 1, 2, 3, 4 or 5 substituents, unless defined otherwise, each of the substituents may be independently selected from the group consisting of hydroxy, fluorine, chlorine, bromine, branched or unbranched $C_{1-4}$-alkoxy, branched or unbranched $C_{1-4}$-perfluoroalkoxy, branched or unbranched $C_{1-4}$ -perfluoroalkyl, amino, carboxy, amido, cyano, nitro, $-SO_2NH_2$, $-CO-C_{1-4}$-alkyl, $-SO-C_{1-4}$-alkyl, $-SO_2-C_{1-4}$-alkyl, $-NH-SO_2-C_{1-4}$-alkyl, wherein the $C_{1-4}$-alkyl may in each case be branched or unbranched, and a phenyl group, more preferably be selected from the group consisting of hydroxy, F, Cl, Br, methoxy, ethoxy, $CF_3$ and a phenyl group.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl.

If any of the residues $R^4$—$R^8$ represents or comprises a linear or branched alkylene group, said alkylene group may preferably be selected from the group consisting of -methylene $-(CH_2)-$, ethylene $-(CH_2-CH_2)-$, n-propylene $-(CH_2-CH_2-CH_2)-$ or isopropylene $-(-C(CH_3)_2)-$.

Preferred are substituted pyrazoline compounds of general formula I given above, wherein

$R^1$ represents an optionally at least mono-substituted phenyl group,

$R^2$ represents an optionally at least mono-substituted phenyl group,

$R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or $R^3$ represents an optionally at least mono-substituted aryl or heteroaryl group, which may be condensed with an optionally at least mono-substituted mono- or polycyclic ring system, or $R^3$ represents an $-NR^4R^5$-moiety,

$R^4$ and $R^5$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group, an $-SO_2-R^6$-moiety, or an $-NR^7R^8$-moiety,

$R^6$ represents a linear or branched, saturated or unsaturated, optionally at least mono-substituted aliphatic group, a saturated or unsaturated, optionally at least mono-substituted, optionally at least one heteroatom as ring member containing cycloaliphatic group, which may be condensed with a mono- or polycyclic ring-system, or an optionally at least mono-substituted aryl or heteroaryl group, which may be condensed with a mono- or polycyclic ring system and/or bonded via a linear or branched alkylene group,

$R^7$ and $R^8$, identical or different, represent a hydrogen atom, an unbranched or branched, saturated or unsaturated, optionally at least mono-substituted aliphatic radical, a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system and/or bonded via a linear or branched alkylene group,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,
whereby preferably the following provisos (disclaimers) apply:

that $R^4$ and $R^5$ do not both represent a hydrogen atom, and that if one of the residues $R^4$ and $R^5$ represents a hydrogen atom or a linear or branched, saturated or unsaturated, substituted or unsubstituted aliphatic group, the other one of these residues $R^4$ and $R^5$ does not represent a substituted or unsubstituted pyridyl group, a substituted or unsubstituted pyrimidyl group, a substituted or unsubstituted pyridazyl group, a substituted or unsubstituted pyrazinyl group, a substituted or unsubstituted thienyl group, a substituted or unsubstituted benzyl group, a substituted or unsubstituted phenethyl group, a substituted or unsubstituted phenyl group, a substituted or unsubstituted phenyl group, which is condensed (attached) to at least one, optionally substituted ring or ringsystem, an —NH-phenyl-moiety, wherein the phenyl group may be at least mono-substituted, an unsubstituted or substituted thiazole group, or an unsubstituted or substituted [1,3,4]thiadiazole group.

Preferred are also substituted pyrazoline compounds of general formula I given above, wherein $R^1$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and -(C=O)-NR'R'', whereby R' and R'' for each substituent independently represent linear or branched $C_{1-6}$ alkyl, preferably $R^1$ represents a phenyl group, which is optionally substituted by one or more substituents selected from the group consisting of methyl, ethyl, F, Cl, Br and $CF_3$, more preferably $R^1$ represents a phenyl group, which is substituted with a chlorine atom in the 4-position, and $R^2$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are substituted pyrazoline compounds of general formula I given above, wherein $R^2$ represents a phenyl group, which is optionally substituted by one or more substituents independently selected from the group consisting of a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-R', SH, SR', SOR', $SO_2R'$, $NH_2$, NHR', NR'R'', -(C=O)-$NH_2$, -(C=O)-NHR' and -(C=O)-NR'R'' whereby R' and R'' for each substituent independently represent linear or branched $C_{1-6}$ alkyl, preferably $R^2$ represents a phenyl group, which is optionally substituted by one or more substituents selected from the group consisting of methyl, ethyl, F, Cl, Br and $CF_3$, more preferably $R^2$ a phenyl group, which is di-substituted with two chlorine atoms in the 2- and 4-position, and $R^1$ and $R^3$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to substituted pyrazoline compounds of general formula I given above, wherein $R^3$ represents a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or $R^3$ represents an optionally at least mono-substituted, 5- or 6-membered aryl or heteroaryl group, which may be condensed with an optionally at least mono-substituted mono- or polycyclic ring system, or $R^3$ represents an -$NR^4R^5$-moiety, preferably $R^3$ represents a saturated, optionally at least mono-substituted, optionally one or more nitrogen-atoms as ring member containing $C_{3-8}$ cycloaliphatic group, which may be condensed with an optionally at least mono-substituted mono- or polycyclic ring system, or $R^3$ represents an —$NR^4R^5$-moiety, more preferably $R^3$ represents a pyrrolidinyl group, a piperidinyl group or a piperazinyl group, whereby each of these groups may be substituted with one or more $C_{1-6}$-alkyl groups, or $R^3$ represents an —$NR^4R^5$-moiety and $R^1$, $R^2$ and $R^4$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Furthermore, substituted pyrazoline compounds of general formula I given above are preferred, wherein $R^4$ and $R^5$, identical or different, represent a hydrogen atom, an unbranched or branched, saturated or unsaturated, optionally at least mono-substituted $C_{1-6}$-aliphatic radical, a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system and/or bonded via a methylene (-$CH_2$-) or ethylene (-$CH_2$-$CH_2$)-group, an —$SO_2$-$R^6$-moiety, or an -$NR^7R^8$-moiety, preferably one of these residues $R^4$ and $R^5$ represents a hydrogen atom and the other one of these residues $R^4$ and $R^5$

represents a saturated or unsaturated, optionally at least mono-substituted, optionally at least one heteroatom as ring member containing $C_{3-8}$-cycloaliphatic group, which may be condensed with an optionally at least mono-substituted mono- or polycyclic ring system, or an optionally at least mono-substituted, 5- or 6-membered aryl or heteroaryl group, which may be condensed with an optionally at least mono-substituted mono- or polycyclic ring system, an —$SO_2$-$R^6$-moiety, or an -$NR^7R^8$-moiety, or $R^4$ and $R^5$, identical or different, each represent a $C_{1-6}$ alkyl group, more preferably one of these residues $R^4$ and $R^5$ represents a hydrogen atom and the other one of these residues $R^4$ and $R^5$ represents an optionally at least mono-substituted pyrrolidinyl group, an optionally at least mono-substituted piperidinyl group, an optionally at least mono-substituted piperazinyl group, an optionally at least mono-substituted triazolyl group, an -$SO_2$-$R^6$-moiety, or an - $NR^7R^8$-moiety, or $R^4$ and $R^5$, identical or different, represent a methyl group, an ethyl group, an n-propyl group, an isopropyl group, an n-butyl group, a sec-butyl group or a tert.-butyl group, and $R^1$-$R^3$ and $R^6$-$R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are substituted pyrazoline compounds of general formula I given above, wherein $R^6$ represents a linear or branched, saturated or unsaturated, optionally at least mono-substituted $C_{1-6}$ aliphatic group, a saturated or unsaturated, optionally at least mono-substituted, optionally at least one heteroatom as ring member containing $C_{3-8}$ cycloaliphatic group, which may be condensed with a mono- or polycyclic ring-system, or an optionally at least mono-substituted, 5- or 6-membered aryl or heteroaryl group, which may be condensed with a mono- or polycyclic ring system and/or bonded via a methylene ($-CH_2-$) or ethylene ($-CH_2-CH_2-$)-group, preferably $R^6$ represents a $C_{1-6}$-alkyl group, a saturated, optionally at least mono-substituted cycloaliphatic group, which may be condensed with a mono- or polycyclic ring-system, or a phenyl group, which is optionally substituted with one or more $C_{1-6}$ alkyl groups, and $R^1$-$R^5$, $R^7$ and $R^8$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Moreover substituted pyrazoline compounds of general formula I given above are preferred, wherein $R^7$ and $R^8$, identical or different, represent a hydrogen atom, an unbranched or branched, saturated or unsaturated, optionally at least mono-substituted $C_{1-6}$ aliphatic radical, a saturated or unsaturated, optionally at least mono-substituted, optionally at least one heteroatom as ring member containing $C_{3-8}$ cycloaliphatic group, which may be condensed with an optionally at least mono-substituted mono- or polycyclic ring system, or an optionally at least mono-substituted, 5- or 6 membered aryl or heteroaryl group, which may be condensed with an optionally at least mono-substituted mono- or polycyclic ring system and/or bonded via a methylene ($-CH_2-$) or ethylene ($-CH_2-CH_2-$)-group, preferably represent a hydrogen atom or a $C_{1-6}$ alkyl radical, and $R^1$-$R^6$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Particularly preferred are compounds of general formula I given below,

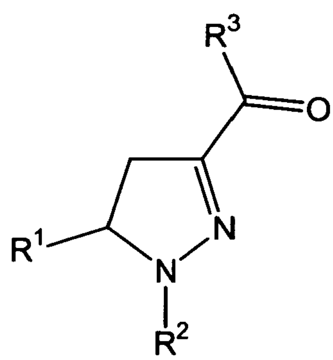

I

wherein

$R^1$ represents a phenyl ring, which is mono-substituted with a halogen atom, preferably a chlorine atom, in its 4-position,

$R^2$ represents a phenyl ring, which is di-substituted with two halogen atoms, preferably chlorine atoms, in its 2- and 4-position,

$R^3$ represents a pyrrolidinyl group, a piperidinyl group, a piperazinyl group, a homo-piperazinyl group, a morpholinyl group, or an $-NR^4R^5$-moiety,

$R^4$ represents a hydrogen atom or a linear or branched $C_{1-6}$-alkyl group,

$R^5$ represents a linear or branched $C_{1-6}$ alkyl group, a pyrrolidinyl group, a piperidinyl group, a piperazinyl group, a homo-piperazinyl group, a morpholinyl group, a triazolyl group, whereby each of the heterocyclic rings may be substituted with one or more, identical or different, $C_{1-6}$-alkyl groups, or an $-SO_2-R^6$-moiety, and

$R^6$ represents a phenyl group, which is optionally substituted with one or more $C_{1-6}$ alkyl groups, which may be identical or different,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Most particularly preferred are substituted pyrazoline compounds selected from the group consisting of:

(Rac)-N-piperidinyl-5-(4-chloro-phenyl)-1-(2,4-dichlorophenyl)-4,5-dihydro-1H-pyrazole-3-carboxamide,

(S-)-N-piperidinyl-5-(4-chloro-phenyl)-1-(2,4-dichlorophenyl)-4,5-dihydro-1H-pyrazole-3-carboxamide,

(R-)-N-piperidinyl-5-(4-chloro-phenyl)-1-(2,4-dichlorophenyl)-4,5-dihydro-1H-pyrazole-3-carboxamide,

5-(4-Chloro-phenyl)-1-(2,4-dichloro-phenyl)-4,5-dihydro-1H-pyrazole-3-carboxylic acid-[1,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone,

N-[5-(4-Chloro-phenyl)-1-(2,4-dichlorophenyl)-4,5-dihydro-1H-pyrazole-3-carbonyl]-4-methylphenylsufonamide,

optionally in the form of a corresponding N-oxide, or a corresponding salt, or a corresponding solvate.

In another aspect the present invention also provides a process for the preparation of substituted pyrazoline compounds of general formula I, according to which at least one benzaldehyde compound of general formula IV

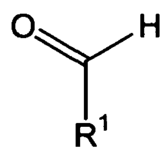

(IV)

wherein $R^1$ has the meaning given above, is reacted with a pyruvate compound of general formula (V)

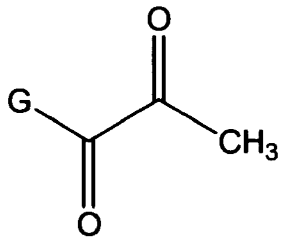

(V),

wherein G represents an OR group with R being a branched or unbranched $C_{1-6}$ alkyl radical, preferably an ethyl radical, or G represents an $O^{-}K$ group with K being a cation, preferably a monovalent cation, more preferably an alkali metal cation, even more preferably a sodium cation, to yield a compound of general formula (VI)

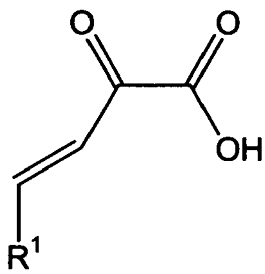

(VI)

wherein $R^1$ has the meaning given above, which is optionally isolated and/or optionally purified, and which is reacted with an optionally substituted phenyl hydrazine of general formula (VII)

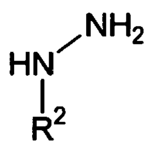

(VII)

or a corresponding salt thereof, wherein $R^2$ has the meaning given above, under an inert atmosphere, to yield a compound of general formula (VIIII)

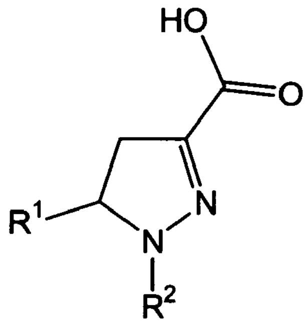

(VIII)

wherein $R^1$ and $R^2$ have the meaning as given above, which is optionally isolated and/or optionally purified, and optionally transferred under inert atmosphere to a compound of general formula (IX)

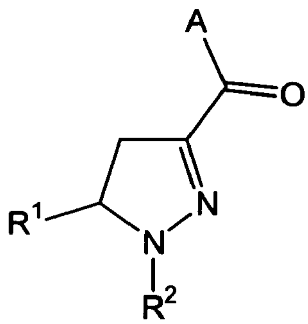

(IX)

wherein the substituents $R^1$ and $R^2$ have the meaning given above and A represents a leaving group, via the reaction with an activating agent, said compound being optionally isolated and/or optionally purified, and at least one compound of general formula (VI) is reacted with a compound of general formula $R^3H$, wherein $R^3$ represents an $-NR^4R^5$-moiety, wherein $R^4$ and $R^5$ have the meaning given above, to yield a substituted pyrazoline compound of general formula I, wherein $R^3$ represents an $-NR^4R^5$-moiety,

and/or at least one compound of general formula (IX) is reacted with a compound of the general formula $R^3H$, in which $R^3$ has the meaning given above to yield a compound of general formula (I) given above, which is optionally isolated and/or optionally purified.

The inventive process is also illustrated in scheme I given below:

Scheme I:

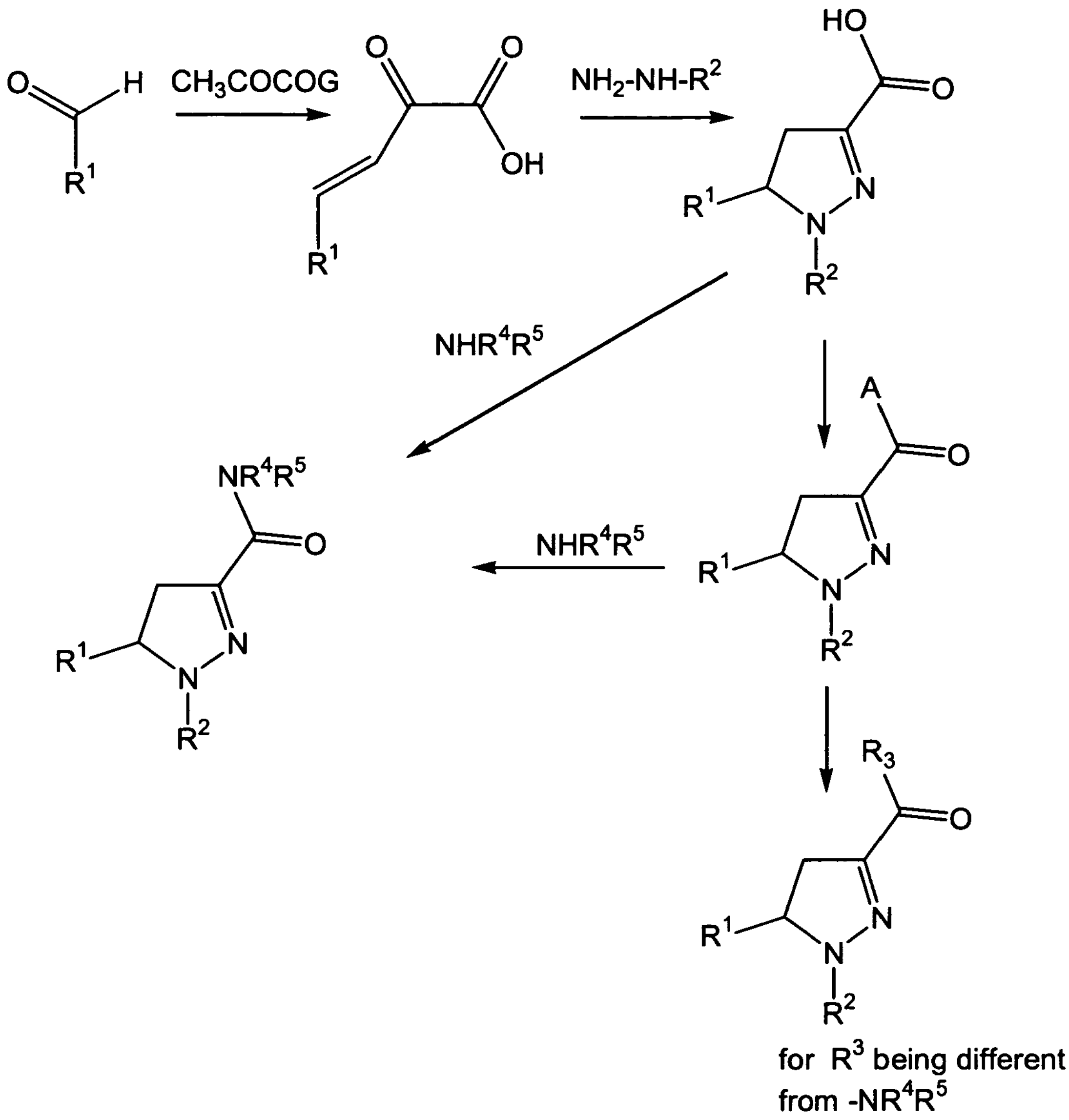


The reaction of the benzaldehyde compound of general formula IV with a pyruvate compound of general formula V is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula IV with a pyruvate compound of general formula V is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (VI) with an optionally substituted phenyl hydrazin of general formula (VII) is preferably carried out in a suitable reaction medium such as $C_{1-4}$-alcohols or ethers such as dioxane or

tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VIII) may be activated for further reactions by the introduction of a suitable leaving group according to conventional methods well known to those skilled in the art. Preferably the compounds of general formula (VIII) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (IX) is an acid chloride, it is preferably prepared by reaction of the corresponding acid of general formula (VIII) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (IX) is a mixed anhydride, said anhydride may preferably be prepared, for example, by reaction of the corresponding acid of general formula (VIII) with ethyl chloroformiate in the presence of a base such as triethylamine or pyridine, in a suitable solvent.

The reaction of general formula (IX) with a compound of general formula $HR^3$ to yield compounds of general formula I, wherein $R^3$ represents an $-NR^4R^5$ moiety is preferably carried out in presence of a base such as triethylamine in a reaction medium such as methylenchloride. The temperature is preferably in the range from 0°C to the boiling point of the reaction medium. The reaction time may vary over a broad range, e.g. from several hours to several days.

The reaction of general formula (IX) with a compound of general formula $HR^3$ to yield compounds of general formula I, wherein $R^3$ represents a saturated or unsaturated, optionally at least mono-substituted, optionally at least one heteroatom as ring member containing cycloaliphatic group, which may be condensed with an optionally at least mono-substituted mono- or polycyclic ring system, or an optionally at least mono-substituted aryl or heteroaryl group, which may be condensed with an optionally at least mono-substituted mono- or polycyclic ring system may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar. The temperature is preferably in teh range from 0°C to the boiling point of the reaction medium, more preferably from 15 to 25 °C. The reaction time may vary over a broad range, e.g. from several minutes to several hours.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

In another aspect the present invention relates to the compound

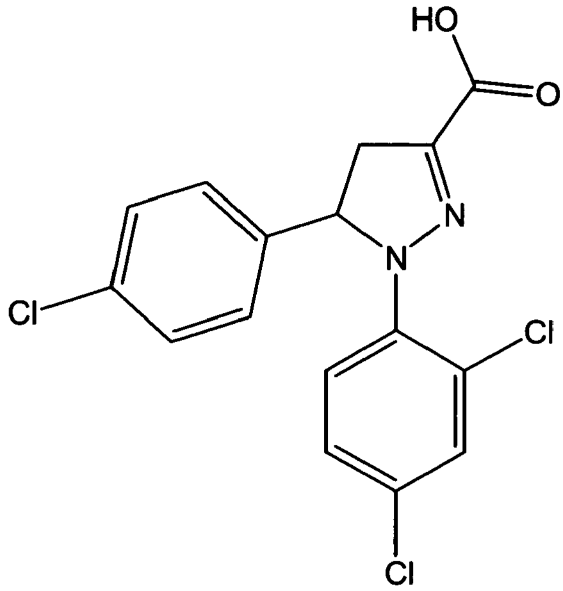

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, in particular as an intermediate in a process for preparing substituted pyrazoline compounds of general formula (I).

In a further aspect the present invention also provides a process for the preparation of salts of substituted pyrazoline compounds of general formula (I) and stereoisomers thereof, wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula (I) or stereoisomers thereof, wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

Solvates, preferably hydrates, of the substituted pyrazoline compounds of general formula (I), of corresponding stereoisomers, of corresponding N-oxides or of corresponding salts thereof may also be obtained by standard procedures known to those skilled in the art.

Substituted pyrazoline compounds of general formula I, which comprise nitrogen-atom containing saturated, unsaturated or aromatic rings may also be obtained in the form of their N-oxides by methods well known to those skilled in the art.

Those skilled in the art understand that the term substituted pyrazoline compounds as used herein is to be understood as encompassing derivatives such as ethers, esters and complexes of these compounds as well. The term "derivatives" as used in this application is defined here as meaning a chemical compound having undergone a chemical derivation starting from an acting (active) compound to change (ameliorate for pharmaceutical use) any of its physico-chemical properties, especially a so-called prodrug, e.g. their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002). The respective description is hereby incorporated by reference and forms part of the disclosure.

The purification and isolation of the inventive substituted pyrazoline compounds of general formula (I), of a corresponding stereoisomer, or salt, or N-oxide, or solvate or any intermediate thereof may, if required, be carried out by conventional methods known to those skilled in the art, e.g. chromatographic methods or recrystallization.

The substituted pyrazoline compounds of general formula (I) given below, their stereoisomers, corresponding N-oxides, corresponding salts thereof and corresponding solvates are toxicologically acceptable and are therefore suitable as pharmaceutical active substances for the preparation of medicaments.

It has been found that the substituted pyrazoline compounds of general formula I given below, stereoisomers thereof, N-oxides thereof, corresponding salts and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these pyrazoline compounds show relatively weak Herg channel affinity, thus a low risk of prolongation of the QT-interval is to be expected for these compounds.

In summary, the inventively used pyrazoline compounds are distinguished by a broad spectrum of beneficial effects, while at the same time showing relatively little undesired effects, i.e. effects which do not positively contribute to or even interfere with the well being of the patient.

Referring to compounds of general formula II,

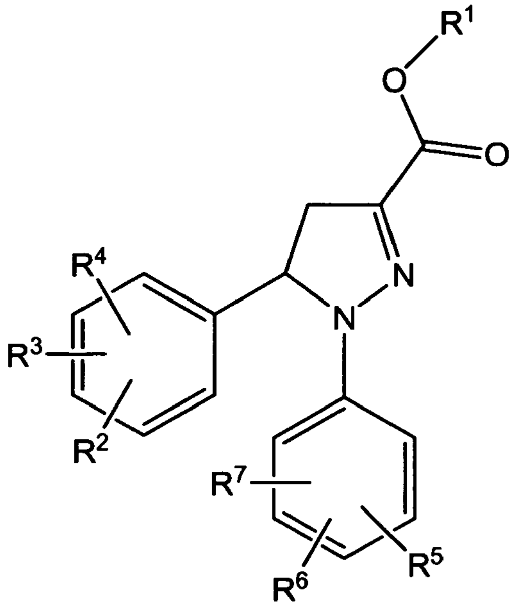

it is very preferred if the following provisio applies:

with the proviso that

if $R^1$ and $R^7$ are H and $R^5$ and $R^6$ both represent Cl in the 3- and 4-position of the phenyl ring neither of $R^2$, $R^3$ and $R^4$ may represent F in the 4-position of the phenyl ring if the other two of $R^2$, $R^3$ and $R^4$ both represent H.

Referring to compounds of general formula II,
preferred linear or branched, saturated or unsaturated aliphatic groups, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl, n-decyl, vinyl, ethinyl, propenyl, propinyl, butenyl and butinyl.

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, $C_{1-2}$-alkyl represents C1- or C2-alkyl, $C_{1-3}$-alkyl represents C1-, C2- or C3-alkyl, $C_{1-4}$-alkyl represents C1-, C2-, C3- or C4-alkyl, $C_{1-5}$-alkyl represents C1-, C2-, C3-, C4-, or C5-alkyl, $C_{1-6}$-alkyl represents C1-, C2-, C3-, C4-, C5- or C6-alkyl, $C_{1-7}$-alkyl represents C1-, C2-, C3-, C4-, C5-, C6- or C7-alkyl, $C_{1-8}$-alkyl represents C1-, C2-, C3-, C4-, C5-, C6-, C7- or C8-alkyl, $C_{1-10}$-alkyl represents C1-, C2-, C3-, C4-, C5-, C6-, C7-, C8-, C9- or C10-

alkyl and $C_{1-18}$-alkyl represents C1-, C2-, C3-, C4-, C5-, C6-, C7-, C8-, C9-, C10-, C11-, C12-, C 13-, C 14-, C15-, C16-, C17- or C18-alkyl. Furthermore, $C_{3-4}$-cycloalkyl represents C3- or C4-cycloalkyl, $C_{3-5}$-cycloalkyl represents C3-, C4- or C5-cycloalkyl, $C_{3-6}$-cycloalkyl represents C3-, C4-, C5- or C6-cycloalkyl, $C_{3-7}$-cycloalkyl represents C3-, C4-, C5-, C6- or C7-cycloalkyl, $C_{3-8}$-cycloalkyl represents C3-, C4-, C5-, C6-, C7- or C8-cycloalkyl, $C_{4-5}$-cycloalkyl represents C4- or C5-cycloalkyl, $C_{4-6}$-cycloalkyl represents C4-, C5- or C6-cycloalkyl, $C_{4-7}$-cycloalkyl represents C4-, C5-, C6- or C7-cycloalkyl, $C_{5-6}$-cycloalkyl represents C5- or C6-cycloalkyl and $C_{5-7}$-cycloalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also $CHF_2$, $CF_3$ or $CH_2OH$) as well as pyrazolinone, oxopyrazolinone, [1,4]-dioxane or dioxolane.

Here, in connection with alkyl and cycloalkyl - unless expressly defined otherwise - the term substituted in the context of this invention is understood as meaning replacement of at least one hydrogen radical by F, Cl, Br, I, $NH_2$, SH or OH, "polysubstituted" radicals being understood as meaning that the replacement takes effect both on different and on the same atoms several times with the same or different substituents, for example three times on the same C atom, as in the case of $CF_3$, or at different places, as in the case of $-CH(OH)-CH=CH-CHCl_2$. Particularly preferred substituents here are F, Cl and OH. In respect of cycloalkyl, the hydrogen radical can also be replaced by $OC_{1-3}$-alkyl or $C_{1-3}$-alkyl (in each case mono- or polysubstituted or unsubstituted), in particular methyl, ethyl, n-propyl, i-propyl, $CF_3$, methoxy or ethoxy.

The term $(CH_2)_{3-6}$ is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2-CH_2-$, $(CH_2)_{1-4}$ is to be understood as meaning $-CH_2-$, $-CH_2-CH_2-$, $-CH_2-CH_2-CH_2-$ and $-CH_2-CH_2-CH_2-CH_2-$, $(CH_2)_{4-5}$ is to be understood as meaning $-CH_2-CH_2-CH_2-CH_2-$ and $-CH_2-CH_2-CH_2-CH_2-CH_2-$, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a $CF_3$, a CN, an $NO_2$, an NRR, a $C_{1-6}$-alkyl (saturated), a $C_{1-6}$-alkoxy, a $C_{3-8}$-cycloalkoxy, a $C_{3-8}$-cycloalkyl or a $C_{2-6}$-alkylene.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound

according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

In a preferred embodiment of the invention for a compound according to formula II at least one of $R^2$, $R^3$ or $R^4$ represents hydrogen, while at least one of $R^2$, $R^3$ or $R^4$ is different from hydrogen.

In a preferred embodiment of the invention for a compound according to formula II $R^7$ represents hydrogen.

In a preferred embodiment of the invention for a compound according to formula II $R^2$, $R^3$ and $R^4$ independently of each other represent hydrogen, a linear or branched $C_{1-6}$-alkyl group, a halogen atom, or $CF_3$, preferably $R^2$, $R^3$ and $R^4$ independently of each other represent hydrogen, methyl, ethyl, F, Cl, Br and $CF_3$.

In a preferred embodiment of the invention for a compound according to formula II $R^5$ and $R^6$ independently of each other represent a linear or branched $C_{1-6}$-alkyl group, a halogen atom, or $CF_3$, preferably $R^5$ and $R^6$ independently of each other represent methyl, ethyl, F, Cl, Br and $CF_3$.

In a preferred embodiment of the invention for a compound according to formula II $R^2$ represents a chlorine atom in the 4-position of the phenyl ring, while $R^3$ and $R^4$ represent hydrogen.

In a preferred embodiment of the invention for a compound according to formula II $R^5$ and $R^6$ each represent a chlorine atoms in the 2- and 4-position of the phenyl ring, while $R^7$ represents hydrogen.

In a preferred embodiment of the invention for a compound according to formula II $R^1$ represents hydrogen, methyl or ethyl, preferably hydrogen.

In a highly preferred further aspect of the invention the compound of general formula II is represented by a compound of general formula III

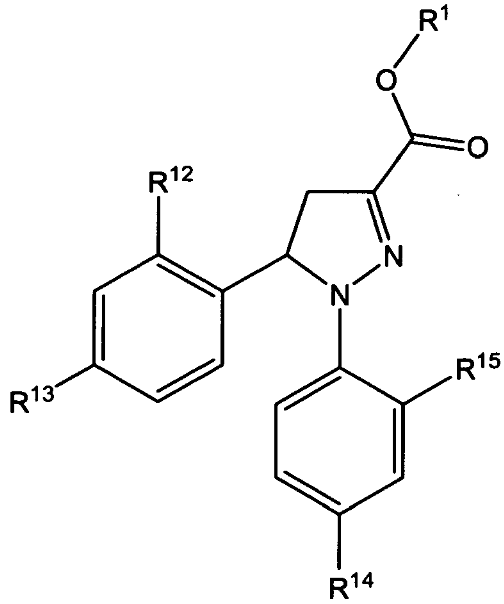

III

wherein

$R^1$ represents hydrogen or a linear or branched $C_{1-4}$-alkyl group,

$R^{12}$ or $R^{13}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,

$R^{14}$ or $R^{15}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

In a preferred embodiment of the invention for a compound according to formula III $R^{12}$ and $R^{13}$ independently of each other represent hydrogen, a linear or branched $C_{1-6}$-alkyl group, a halogen atom, or $CF_3$, preferably $R^{12}$ and $R^{13}$ independently of each other represent hydrogen, methyl, ethyl, F, Cl, Br and $CF_3$.

In a preferred embodiment of the invention for a compound according to formula III $R^{14}$, and $R^{15}$ independently of each other represent a linear or branched $C_{1-6}$-alkyl group, a halogen atom, or $CF_3$, preferably $R^{14}$ and $R^{15}$ independently of each other represent methyl, ethyl, F, Cl, Br and $CF_3$.

In a preferred embodiment of the invention for a compound according to formula III $R^{13}$ represents Cl and $R^{12}$ represents hydrogen.

In a preferred embodiment of the invention for a compound according to formula III $R^{14}$ and $R^{15}$ each represent Cl.

In a preferred embodiment of the invention for a compound according to formula III $R^1$ represents hydrogen, methyl or ethyl, preferably hydrogen.

In another preferred embodiment the compound according to formula II or III is selected from the group consisting of:

(Rac-) 5-(4-chloro-phenyl)-1-(2,4-dichlorophenyl)-4,5-dihydro-1H-pyrazol-3-carboxylic acid,

(S-) 5-(4-chloro-phenyl)-1-(2,4-dichlorophenyl)-4,5-dihydro-1H-pyrazol-3-carboxylic acid,

(R-) 5-(4-chloro-phenyl)-1-(2,4-dichlorophenyl)-4,5-dihydro-1H-pyrazol-3-carboxylic acid,

optionally in the form of a corresponding N-oxide, a corresponding salt or a corresponding solvate.

Another preferred embodiment of the invention covers also any prodrug of the compounds of the invention described above as well as any medicament comprising this and any use thereof; especially including their esters and ethers. Examples of well known methods of producing a prodrug of a given acting compound are known to those skilled in the art and can be found e.g. in Krogsgaard-Larsen et al., Textbook of Drugdesign and Discovery, Taylor & Francis (April 2002).

In another aspect the present invention also provides a process for the preparation of substituted pyrazoline compounds of general formula II or III, wherein $R^1$ is hydrogen, given above, in that at least one benzaldehyde compound of general

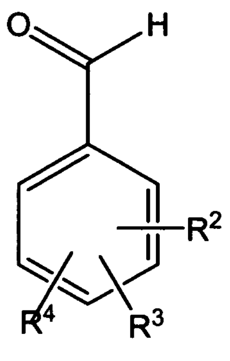

formula IV'

(IV')

wherein $R^2$, $R^3$ and $R^4$ have the meaning mentioned above, is reacted with a pyruvate compound of general formula (V')

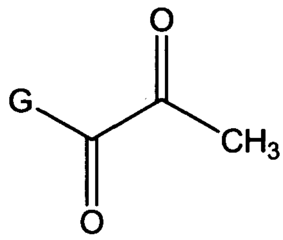

(V'),

wherein G represents an OR group with R being a branched or unbranched $C_{1-6}$ alkyl radical or G represents an O-K group with K being a cation, preferably an anorganic kation, more preferably an alkali metal kation, most preferably sodium, to yield a compound of general formula (VI')

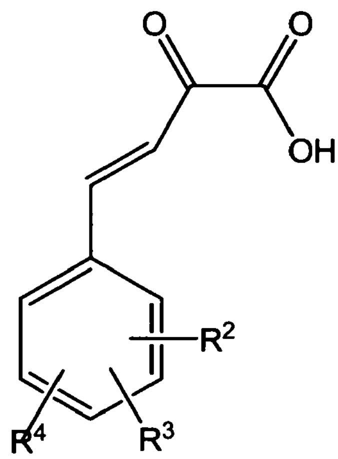

(VI')

which is optionally isolated and/or optionally purified, and which is reacted with an optionally substituted phenyl hydrazine of general formula (VII')

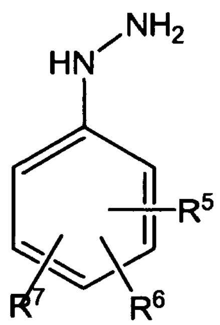

(VII')

or a corresponding salt thereof, wherein $R^5$, $R^6$ and $R^7$ have the meaning mentioned above, under inert atmosphere,

to yield a compound of general formula (VIII')

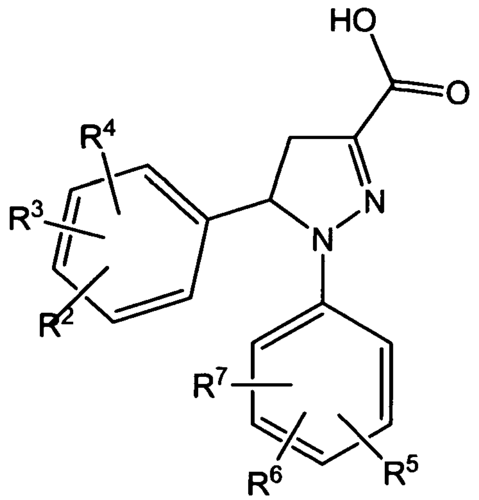

(VIII')

wherein $R^2$, $R^3$, $R^4$, $R^5$, $R^6$ and $R^7$ have the meaning as given above, which is optionally isolated and/or optionally purified, and optionally esterified to an alkyl-ester if in the substituted pyrazoline compound of general formula I or II according to the invention $R^1$ is a linear or branched $C_{1-4}$-alkyl group.

The inventive process is also illustrated in scheme I' given below:

Scheme I':

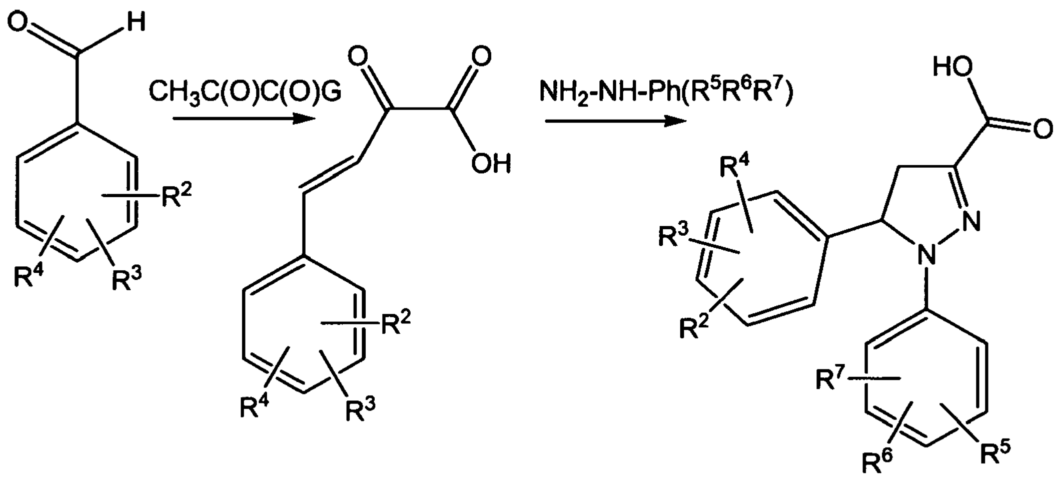


The reaction of the benzaldehyde compound of general formula III with a pyruvate compound of general formula V' is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably said reaction is carried out in a protic reaction medium such as a $C_{1-4}$ alkyl alcohol or mixtures of these.

Reaction temperature as well as the duration of the reaction may vary over a broad range. Preferred reaction

temperatures range from -10 °C to the boiling point of the reaction medium. Suitable reaction times may vary for example from several minutes to several hours.

Also preferred the reaction of the benzaldehyde compound of general formula III with a pyruvate compound of general formula V' is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (VI') with an optionally substituted phenyl hydrazin of general formula (VII') is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VIII') may be activated for further reactions by the introduction of a suitable leaving group according to conventional methods well known to those skilled in the art. Preferably the compounds of general formula (VIII') are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (VIII') is an acid chloride, it is preferably prepared by reaction of the corresponding acid of general formula (VIII') with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (VIII') is a mixed anhydride, said anhydride may preferably be prepared, for example, by reaction of the corresponding acid of general formula (VIII') with ethyl chloroformiate in the presence of a base such as triethylamine or pyridine, in a suitable solvent.

Following that the activated compound can be reacted with an alkyl-alcohol to arrive at compounds according to general formulas II or III with $R^1$ being a a linear or branched $C_{1-4}$-alkyl group.

During the processes described above the protection of sensitive groups or of reagents may be necessary and/or desirable. The introduction of conventional protective groups as well as their removal may be performed by methods well-known to those skilled in the art.

If the substituted pyrazoline compounds of general formula II or III themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. It is also possible to obtain pure stereoisomers via stereoselective synthesis.

In a further aspect the present invention also provides a process for the preparation of salts of substituted pyrazoline compounds of general formula II or III and stereoisomers thereof, wherein at least one compound of general formula II or III having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted pyrazoline compounds of general formula II or III or stereoisomers thereof, wherein at least one compound of general formula II or III having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

Solvates, preferably hydrates, of the substituted pyrazoline compounds of general formula II or III, of corresponding stereoisomers, of corresponding N-oxides or of corresponding salts thereof may also be obtained by standard procedures known to those skilled in the art.

Substituted pyrazoline compounds of general formula II or III, which comprise nitrogen-atom containing saturated, unsaturated or aromatic rings may also be obtained in the form of their N-oxides by methods well known to those skilled in the art.

The purification and isolation of the inventive substituted pyrazoline compounds of general formula II or III, of a corresponding stereoisomer, or salt, or solvate or any intermediate thereof may, if required, be carried out by conventional methods known to those skilled in the art, e.g. chromatographic methods or recrystallization.

In a preferred embodiment of the combination of compounds according to the invention the compound according to formula II or III is selected from the group consisting of:

5-(4-chloro-phenyl)-1-(2,4-dichlorophenyl)-4,5-dihydro-1H-pyrazol-3-carboxylic acid,

(Rac-)5-(4-chloro-phenyl)-1-(2,4-dichlorophenyl)-4,5-dihydro-1H-pyrazol-3-carboxylic acid,

(S-)5-(4-chloro-phenyl)-1-(2,4-dichlorophenyl)-4,5-dihydro-1H-pyrazol-3-carboxylic acid,

(R-)5-(4-chloro-phenyl)-1-(2,4-dichlorophenyl)-4,5-dihydro-1H-pyrazol-3-carboxylic acid,

optionally in the form of a corresponding N-oxide, a corresponding salt or a corresponding solvate.

In a preferred embodiment of the combination of compounds according to the invention the combination of compounds comprises at least one compound according to formula I selected from the group consisting of:

(Rac-)piperidinyl-5-(4-chloro-phenyl)-1-(2,4-dichlorophenyl)-4,5-dihydro-1H-pyrazol-3-carboxamide,

(R-)piperidinyl-5-(4-chloro-phenyl)-1-(2,4-dichlorophenyl)-4,5-dihydro-1H-pyrazol-3-carboxamide,

(S-)piperidinyl-5-(4-chloro-phenyl)-1-(2,4-dichlorophenyl)-4,5-dihydro-1H-pyrazol-3-carboxamide,

5-(4-Chloro-phenyl)-1-(2,4-dichloro-phenyl)-4,5-dihydro-1H-pyrazole-3-carboxylic acid-[1 ,2,4]-triazole-4-yl-amide,

5-(4-Chloro-phenyl)-1-(2,4-dichloro-phenyl)-4,5-dihydro-1H-pyrazole-3-carboxylic acid-(4-methyl-piperazin-1-yl)-amide,

5-(4-Chloro-phenyl)-1-(2,4-dichloro-phenyl)-4,5-dihydro-1H-pyrazole-3-carboxylic acid diethylamide,

[5-(4-Chloro-phenyl)-1-(2,4-dichloro-phenyl)-4,5-dihydro-1H-pyrazole-3-yl]-piperidine-1-yl-methanone,

N-[5-(4-Chloro-phenyl)-1-(2,4-dichlorophenyl)-4,5-dihydro-1H-pyrazole-3-carbonyl]-4-methylphenylsulfonamide,

optionally in the form of a corresponding N-oxide, a corresponding salt or a corresponding solvate.

In a preferred embodiment of the combination of compounds according to the invention the combination of compounds comprises at least one compound according to formula I selected from
5-(4-chloro-phenyl)-1-(2,4-dichlorophenyl)-4,5-dihydro-1 H-pyrazol-3-carboxylic acid,
and
N-piperidinyl-5-(4-chloro-phenyl)-1-(2,4-dichlorophenyl)-4,5-dihydro-1H-pyrazol-3-carboxamide;
each optionally in the form of a corresponding N-oxide, a corresponding salt or a corresponding solvate.

The term "derivatives" as used in this application is defined here as meaning a chemical compound having undergone a chemical derivation such as substitution or addition of a further chemical group to change (for pharmaceutical use) any of its physico-chemical properties, such as solubility or bioavailability. Derivatives include so-called prodrugs, e.g. ester and ether derivatives of an active compound that yield the active compound per se after administration to a subject.

Examples of well known methods of producing a prodrug of a given acting compound are known to those skilled in the art and can be found e.g. in Krogsgaard-Larsen et al., Textbook of Drugdesign and Discovery, Taylor & Francis (April 2002).

The term "drug-induced obesity" refers to the gain of weight during or after treatment of an individual with at least one compound/medicament/substance.

Diseases associated with the cannabinoid receptor system, as defined in the present invention, comprise acute pain, allodynia, analgesia, angina pain, bone injury pain, cancer pain, central neuropathic pain, central pain, chronic lower back pain, chronic pain, cluster headaches, dental pain, gastrointestinal pain, genitourinary tract-related pain,

herpes neuralgia, HIV/AIDS-related pain, inflammatory pain, metabolic neuropathies, neuropathic pain, nociception, nociceptive pain, non-central neuropathic pain, pain associated with de-addiction of drugs, pain associated with spinal cord injury, pain during labor and delivery, pain from cystitis, pain from vascular lesions in the brain, pain resulting from burns, pain resulting from sunburns, pelvic pain, phantom limb pain, post partum pains, post-operative pain, post-stroke pain, Reflex sympathetic dystrophy (RSD), resistant pain, surgical pain, trigeminal pain, visceral pain, amenorrhea, dysmenorrhea, endometriosis, erectile dysfunction, impotence, infertility, menorrhagia, menstrual disorders, premature ejaculation, premature menopause, sexual dysfunction, vaginitis, vulvitis, achalasia, acute or chronic pancreatitis, atrophy of gastric glands, Barrett's metaplasia, carcinoid tumors, chronic erosive gastritis, chronic inflammatory diseases of the bowel, chronic intestinal pseudo-obstruction, colonic inertia, constipation, cricopharyngeal incoordination, Crohn's disease, cystadenocarcinoma, diffuse esophageal spasm, ductal adenocarcinoma, duodenal ulcers, dysphagia, emesis, familial polyposis, functional dyspepsia, gastric ulcers, gastrinoma, gastritis, gastroesophageal reflux, globus sensation, glucagonoma, inflammation of the gastric mucosa, insulinoma, irritable bowel syndrome, islet cell tumors, malabsorption syndrome, megacolon, metaplasia of gastric tissues, multiple endocrine neoplasia syndrome, nausea, neoplasms of the stomach, polyps, pre-esophageal dysphagia, steatorrhea, stress gastritis, ulcerative colitis, vigoruos achalasia, Vipoma syndrome, Zollinger-Ellison syndrome, age-associated memory impairment, age-related cognitive decline, attention deficit disorders, attention deficit hyperactivity disorders, brain injuries, catalepsy, cerebral apoplexy, cerebral ischemia, cerebral vascular accidents, corticobasal degeneration, Creutzfeld Jakob dementia, dementia, dementia with Lewy bodies, frontotemporal dementia, HIV dementia, Korsakoff s psychosis, learning disabilities, memory deficits, mild cognitive impairment, motor neuron disease, multiple sclerosis, neuropathy, Parkinsonism linked to chromosome 17, Pick's disease, post-stroke, post-traumatic brain injury, progressive nuclear palsy, schizophrenia with dementia, small-vessel cerebrovascular disease, thalamic degeneration, traumatic brain injury, vascular cognitive impairment, vascular dementia, atherosclerosis, atrial and ventricular arrhythmias, cardiac insufficiency, congestive heart failure, hypertension, hypertensive vascular diseases, ischemic diseases of the heart, myocardial infarction, peripheral vascular diseases, appetence, appetite disorders, metabolic syndrome, diabetes type I, drug-induced obesity, eating disorders assoc. with excessive food intake, Guillain-Barre syndrome, acute or chronic hepatitis, alpha-antitrypsin-deficiency, ascites, benign neoplasms of the liver, Budd-Chiari syndrome, chronic cholestatic liver disease, cirrhosis of the liver, Crigler-Najjar syndrome, drug-induced disorders of the liver, Dubin-Johnson syndrome, fatty liver, Gaucher's syndrome, Gilbert's syndrome, hepatic granulomas, hepatomegaly, inflammatory conditions of the liver due to viruses, bacteria, fungi, protozoa or helminths, intrahepatic cholestasis, jaundice, liver fibrosis, malignant neoplasms of the liver, portal hypertension, portal-systemicencephalopathy, postoperative intrahepatic cholestasis, primary biliary cirrhosis, primary sclerosing cholangitis, Reye's syndrome, Rotor syndrome, steatosis, viral hepatitis, Wilson's syndrome, convulsions, demyelinisation related disorders, medicament-induced movement disorders, seizures, spinal cord injury, septic shock, viral encephalitis, adenocarcinomas, benign neoplasms, brain tumors, cancer of adipous tissue, cancer of blood vessel, cancer of cartilage tissue, cancer of connective tissue, cancer of muscle tissue, cancer of the endocrine glands, cancer of the gastrointestinal tract, cancer of the penis, cancer of the respiratory tract, cancer of the urogenital system, cancers of blood-forming tissue, carcinoma, carcinosarcoma, dysplasias, hyperplasias, larynx cancer, leukemias, lymphomas, metastasis, metastatic tumors, neck cancer, neoplasms, pituitary cancer, rectum cancer, sarcoma, small intestine, spleen cancer, testes cancer, thyroid cancer, tongue cancer, tumors of nerve tissues, uterus cancer, stress, sedation and spinal cord injury.

In a preferred aspect of the present invention, disclaimed diseases associated with the cannabinoid receptor system, as defined in the present invention, include migraine, potentiation of analgesics, diarrhea, intestinal motility disorders, intestinal transit, vomiting, Alzheimer's disease, anxiety, cerebellar disorders, craniocerebral trauma, stroke, depression, epilepsy, head trauma, Huntington's disease, insomnia, panic attacks, peripheric neuropathy, psychosis, schizophrenia, senile dementia, stroke panic attacks, substance abuse disorders, hemorragic shock, hypotension, Raynaud's syndrome, sclerotic plaques, Food intake disorders, bulimia, cachexia, obesity, diabetes type II, asthma, cancer-associated bone disease, osteoporosis, Paget's disease of bone, alcohol abuse, alcohol addiction, drug abuse, drug addiction, medicament abuse, medicament addiction, nicotine abuse, nicotine addiction, dystonia, Parkinson's disease, tardive dyskinesia, tremor, endotoxemic shock, bladder cancer, bone cancer, bowel cancer, brain cancer, breast cancer, cervical cancer, colon cancer, esophageal cancer, lip cancer, liver cancer, lung cancer, mouth cancer, ovary cancer, pancreas cancer, prostate cancer, skin cancer, stomach cancer, glaucoma, hypothermia, pruritus and thymic disorders.

A preferred aspect of the present invention is the use of substituted pyrazoline compounds of the general formula I, II, III and X as mentioned above, as well as combinations of substituted pyrazoline compounds of the general formula I, II, III and/or X and/or of one of its/their derivatives, optionally in the form of its/their racemate, pure stereoisomers, especially enantiomers or diastereomers or in the form of mixtures of stereoisomers, especially enantiomers or diastereomers; in any suitable ratio; in neutral form, in the form of an acid or base or in form of a salt, especially a physiologically acceptable salt, or in form of a solvate, especially a hydrate,
for the production of a drug for the treatment of pain, allodynia, analgesia, angina pain, bone injury pain, cancer pain, central neuropathic pain, central pain, chronic lower back pain, chronic pain, cluster headaches, dental pain, gastrointestinal pain, genitourinary tract-related pain, herpes neuralgia, HIV/AIDS-related pain, inflammatory pain, metabolic neuropathies, neuropathic pain, nociception, nociceptive pain, non-central neuropathic pain, pain associated with de-addiction of drugs, pain associated with spinal cord injury, pain during labor and delivery, pain from cystitis, pain from vascular lesions in the brain, pain resulting from burns, pain resulting from sunburns, pelvic pain, phantom limb pain, post partum pains, post-operative pain, post-stroke pain, Reflex sympathetic dystrophy (RSD), resistant pain, surgical pain, trigeminal pain, visceral pain, amenorrhea, dysmenorrhea, endometriosis, erectile dysfunction, impotence, infertility, menorrhagia, menstrual disorders, premature ejaculation, premature menopause, sexual dysfunction, vaginitis, vulvitis, achalasia, acute or chronic pancreatitis, atrophy of gastric glands, Barrett's metaplasia, carcinoid tumors, chronic erosive gastritis, chronic inflammatory diseases of the bowel, chronic intestinal pseudo-obstruction, colonic inertia, constipation, cricopharyngeal incoordination, Crohn's disease, cystadenocarcinoma, diffuse esophageal spasm, ductal adenocarcinoma, duodenal ulcers, dysphagia, emesis, familial polyposis, functional dyspepsia, gastric ulcers, gastrinoma, gastritis, gastroesophageal reflux, globus sensation, glucagonoma, inflammation of the gastric mucosa, insulinoma, irritable bowel syndrome, islet cell tumors, malabsorption syndrome, megacolon, metaplasia of gastric tissues, multiple endocrine neoplasia syndrome, nausea, neoplasms of the stomach, polyps, pre-esophageal dysphagia, steatorrhea, stress gastritis, ulcerative colitis, vigoruos achalasia, Vipoma syndrome, Zollinger-Ellison syndrome, age-associated memory impairment, age-related cognitive decline, attention deficit disorders, attention deficit hyperactivity disorders, brain injuries, catalepsy, cerebral apoplexy, cerebral ischemia, cerebral vascular accidents, corticobasal degeneration, Creutzfeld Jakob dementia, dementia, dementia with Lewy bodies, frontotemporal dementia, HIV dementia, Korsakoff s psychosis, learning disabilities, memory deficits, mild cognitive impairment, motor neuron disease, multiple sclerosis, neuropathy, Parkinsonism linked to chromosome 17, Pick's disease, post-stroke, post-traumatic brain injury, progressive nuclear palsy, schizophrenia with dementia, small-vessel cerebrovascular disease, thalamic degeneration, traumatic brain injury, vascular cognitive impairment, vascular dementia, atherosclerosis, atrial and ventricular arrhythmias, cardiac insufficiency, congestive heart failure, hypertension, hypertensive vascular diseases, ischemic diseases of the heart, myocardial infarction, peripheral vascular diseases, appetence, appetite disorders, metabolic syndrome, diabetes type I, eating disorders assoc. with excessive food intake, Guillain-Barre syndrome, acute or chronic hepatitis, alpha-antitrypsin-deficiency, ascites, benign neoplasms of the liver, Budd-Chiari syndrome, chronic cholestatic liver disease, cirrhosis of the liver, Crigler-Najjar syndrome, drug-induced disorders of the liver, Dubin-Johnson syndrome, fatty liver, Gaucher's syndrome, Gilbert's syndrome, hepatic granulomas, hepatomegaly, inflammatory conditions of the liver due to viruses, bacteria, fungi, protozoa or helminths, intrahepatic cholestasis, jaundice, liver fibrosis, malignant neoplasms of the liver, portal hypertension, portal-systemicencephalopathy, postoperative intrahepatic cholestasis, primary biliary cirrhosis, primary sclerosing cholangitis, Reye's syndrome, Rotor syndrome, steatosis, viral hepatitis, Wilson's syndrome, convulsions, demyelinisation related disorders, medicament-induced movement disorders, seizures, spinal cord injury, septic shock, viral encephalitis, adenocarcinomas, benign neoplasms, brain tumors, cancer of adipous tissue, cancer of blood vessel, cancer of cartilage tissue, cancer of connective tissue, cancer of muscle tissue, cancer of the endocrine glands, cancer of the gastrointestinal tract, cancer of the penis, cancer of the respiratory tract, cancer of the urogenital system, cancers of blood-forming tissue, carcinoma, carcinosarcoma, dysplasias, hyperplasias, larynx cancer, leukemias, lymphomas, metastasis, metastatic tumors, neck cancer, neoplasms, pituitary cancer, rectum cancer, sarcoma, small intestine, spleen cancer, testes cancer, thyroid cancer, tongue cancer, tumors of nerve tissues, uterus cancer, stress, sedation and spinal cord injury.

In a preferred aspect of the present invention, diseases associated with the cannabinoid receptor system such as migraine, potentiation of analgesics, diarrhea, intestinal motility disorders, intestinal transit, vomiting, Alzheimer's disease, anxiety, cerebellar disorders, craniocerebral trauma, stroke, depression, epilepsy, head trauma, Huntington's disease, insomnia, panic attacks, peripheric neuropathy, psychosis, schizophrenia, senile dementia, stroke panic attacks, substance abuse disorders, hemorragic shock, hypotension, Raynaud's syndrome, sclerotic plaques, Food intake disorders, bulimia, cachexia, obesity, diabetes type II, asthma, cancer-associated bone disease, osteoporosis, Paget's disease of bone, alcohol abuse, alcohol addiction, drug abuse, drug addiction, medicament abuse, medicament addiction, nicotine abuse, nicotine addiction, dystonia, Parkinson's disease, tardive dyskinesia, tremor, endotoxemic shock, bladder cancer, bone cancer, bowel cancer, brain cancer, breast cancer, cervical cancer, colon cancer, esophageal cancer, lip cancer, liver cancer, lung cancer, mouth cancer, ovary cancer, pancreas cancer, prostate cancer, skin cancer, stomach cancer, glaucoma, hypothermia, pruritus and thymic disorders are disclaimed.

Pharmacological Methods

**I. In-vitro determination of affinity to CB1/CB2-Receptors**

The in-vitro determination of the affinity of the inventive substituted pyrazoline compounds to $CB_1$/$CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

**II. In-vivo bioassay system for determination of cannabinoid activity**

**Mouse tetrad model**

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.

Before testing in the behavioral procedures given below, mice are acclimatized to the experimental setting. Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured.

Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 $\pm$ 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula :

$$\% \text{ MPE of Analgesia} = (PT - B) / (PC - B) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective

description is hereby incorporated by reference and forms part of the present disclosure.

The chosen scoring system is

0: no ataxia;

1: doubful;

2: obvious calmness and quiet;

3 pronounced ataxia;

prior to as well as after treatment.

The percentage of sedation is determined according to the formula:

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥-2 °C are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:

more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \times 100$$

**III. In vivo testing for antiobesic activity**

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

## IV. In vivo testing for antidepressant activity

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

The present invention is illustrated below with the aid of examples. These illustrations are given solely by way of example and do not limit the general spirit of the present invention.

The compound according to example 0 is an inhibitor of high blood levels of triglicerides. This effect has been probed in obese mice fed with high fat diet. In the following paragraphs it is described the method and the results obtained in this study.

## V. In-vivo testing for regulation of triglycerides in blood plasma

The study was done using six weeks old male mice B6 Lep ob/ob, obtained from Charles River (France). Mice were divided in 3 groups : I (control), II (vehicle), III (example 0).

Group I:

The animals of the group I received the standard diet (D-12450B, Research Diets, NJ, USA).

Group II:

The animals of the groups II and III were fed with a High Fat Diet (D-12492, Research Diets, NJ, USA), in both cases for 7 weeks (References 1 and 2).

Group III:

The animals of the groups III were fed with a High Fat Diet (D-12492, Research Diets, NJ, USA), in both cases for 7 weeks (References 1 and 2).

At the end of the feeding period of 7 weeks, it was started the treatment period (14 days): Group II mice received the vehicle (10 ml/kg/day, po, of the aqueous solution of acacia gum, 5% W/V). Group III was administered with 30 mg/kg/day, po, of the inventive compound 5-(4-chlorophenyl)-1-(2,4-dichlorophenyl)-4,5-dihydro-pyrazole-3-carboxylic acid according to Example 0. Group I didn't received any treatment. The three groups of mice had the same diet than in the previous period.

At the end of the 14 days period of treatment, the blood levels of triglicerides of the animals were determined.

The analysis of the whole blood samples was done using test strips "Lipid panel" and the photometric Analyzer Cardio-Check Test System, from PA Instruments Polymer Technology Systems Indianapolis, IN-46268, USA (Distributed in Spain by Novalab Iberica S.A.L, Madrid, Spain).

## VI. In-vivo testing for regulation of triglycerides in blood plasma

In a second set of experiments carried out similar to the tests shown above the TG (triglyceride) levels of diet-induced obese mice in blood were determined.

Mice receiving a high fat diet were - after a feeding period of 6 days - either treated p.o. with vehicle (0,5 % HPMC) or with the compound according to example 0 (30 mg/kg/day p.o.).

TG levels in blood were determined on day 28 after beginning of the treatment.

## Examples:

### Example 1:

N-piperidinyl-5-(4-chlorophenyl)-1-(2,4-dichlorophenyl)-4,5-dihydro-1H-pyrazole-3-carboxamide

a) 4-(4-chlorophenyl)-2-oxo-3-butenoic acid

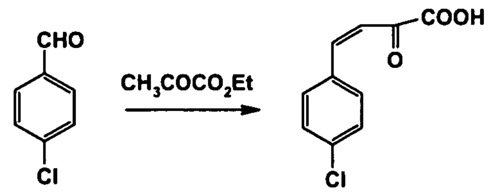


In a three neck flask p-chlorobenzaldehyde (13,3 g, 95 mmoles) and ethyl pyruvate (10 g, 86 mmoles) were dissolved in 150 ml of absolute ethanol. The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$): 3500-2500, 1719,3, 1686,5, 1603,4, 1587,8, 1081,9.

$^1H$ NMR($CDCl_3$, δ): 7,4 (d, J=8,4Hz, 2H), 7,5 (d, J=16,1Hz, 1H), 7,6 (d, J=8,4Hz, 2H), 8,1(d, J=16,1Hz, 1H).

b) 5-(4-chlorophenyl)-1-(2,4-dichlorophenyl)-4,5-dihydro-pyrazole-3-carboxylic acid

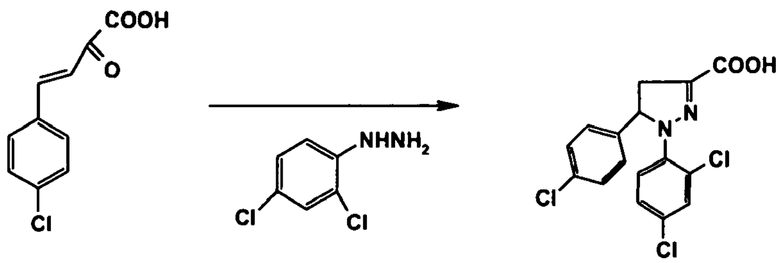

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, $cm^{-1}$): 3200-2200, 1668,4, 1458, 1251,4, 1104,8.

$^1H$ NMR ($CDCl_3$, δ) : 3,3 (dd, 1H), 3,7 (dd, 1 H), 5,9 (dd, 1H), 7,09-7,25 (m, 7H).

(c) 5-(4-chlorophenyl)-1-(2,4-dichlorophenyl)-4,5-dihydro-pyrazole-3-carboxylic acid chloride

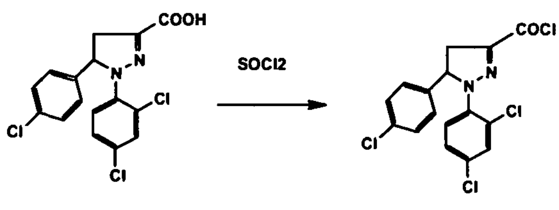


Under nitrogen atmosphere 5-(4-chlorophenyl)-1-(2,4-dichlorophenyl)-4,5-dihydro-pyrazole-3-carboxylic acid

(2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, $cm^{-1}$) : 1732,3, 1700, 1533,3, 1478,1, 1212,9, 826,6.

d) N-piperidinyl-5-(4-chlorophenyl)-1-(2,4-dichlorophenyl)-4,5-dihydropyrazole-3-carboxamide [this compound may also be referred to as 5-(4-Chloro-phenyl)-1-(2,4-dichloro-phenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide]

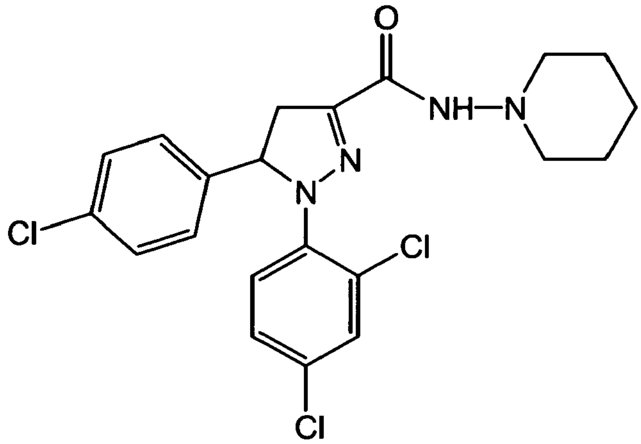

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, $cm^{-1}$) : 3222,9, 2934,9, 1647,4, 1474,7, 1268,3, 815,6.

$^1H$ NMR ($CDCl_3$, δ) : 1,4 (m, 2H), 1,7 (m, 4H), 2,8 (m, 4H), 3,3 (dd, J=6,1 y 18,3Hz, 1H), 3,7 (dd, J=12,5 and 18,3 Hz, 1 H), 5,7 (dd, J=6,1 and 12,5 Hz, 1 H), 7,0-7,2 (m, 6H), 7,4 (s, 1 H).

The compounds according to the following examples 2-6 have been prepared analogously to the process described in Example 1.

**Example 2:**

5-(4-Chloro-phenyl)-1-(2,4-dichloro-phenyl)-4,5-dihydro-1H-pyrazole-3-carboxylic acid-[1,2,4]triazol-4-yl amide

Melting point: 134-138 °C.

IR (KBr, $cm^{-1}$): 3448, 1686, 1477, 1243, 1091, 821.

$^1H$ NMR($CDCl_3$, δ): 3,1 (dd, J=6,2 and 17,9Hz, 1 H), 3,7 (dd, J=12,3 and 17,9Hz, 1 H), 5,9 (dd, J=6,2 and 12,3 Hz, 1H), 7,2-7,5 (m, 7H), 8,7 (s, 2H), 12,0 (bs, 1 H).

**Example 3:**

5-(4-Chloro-phenyl)-1-(2,4-dichloro-phenyl)-4,5-dihydro-1H-pyrazole-3-carboxylicacid-(4-methyl-piperazin-1-yl)-amide hydrochloride

Melting point: 150-155°C.
IR (KBr, $cm^{-1}$): 3433, 1685, 1477, 1296, 1246, 1088, 1014, 825.
$^1H$ NMR ($CDCl_3$, δ): 2,7 (d, J=4,2Hz, 3H), 3,0-3,4 (m, 9H), 3,6 (dd, J=11,9 and 17,9 Hz, 1 H), 5,8 (dd, J=5,5 and 11,9 Hz, 1 H), 7,1 (d, J=8,4Hz, 2H), 7,25 (2d, J= 8,4 and 8,7 Hz, 3H), 7,4 (d, J=2,2Hz, 1 H), 7,5 (d, J=8,7Hz, 1 H), 9,8 (s, 1 H), 11,2 (bs).

**Example 4:**

5-(4-Chloro-phenyl)-1-(2,4-dichloro-phenyl)-4,5-dihydro-1H-pyrazole-3-carboxylic acid diethylamide

This compound was obtained in form of an oil.
IR (film, $cm^{-1}$) : 2974, 1621, 1471, 1274, 1092, 820.
$^1H$ NMR ($CDCl_3$, δ): 1,2 (m, 6H), 3,3-3,9 (m, 6H), 5,6 (dd, J=5,8 and 11,7 Hz, 1 H), 7-7,25 (m, 7H).

**Example 5:**

[5-(4-Chloro-phenyl)-1-(2,4-dichloro-phenyl)-4,5-dihydro-1H-pyrazol-3-yl]-piperidin-1-yl-methanone

Melting point: 105-110°C.
IR (KBr, $cm^{-1}$) : 2934, 1622, 1470, 1446, 1266, 1010, 817.
$^1H$ NMR ($CDCl_3$, δ): 1,7 (m, 6H), 3,4 (dd, J=5,7 and 17,9Hz, 1H), 3,7 (m, 3H), 3,9 (m, 2H), 5,6 (dd, J=6,1 y 11,9 Hz, 1H), 7-7,25 (m, 7H).

**Example 6:**

N-[5-(4-Chloro-phenyl)-1-(2,4-dichloro-phenyl)-4,5-dihydro-1H-pyrazole-3-carbonyl]-4-methyl-phenylsulfonamide

This compound was obtained in form of an amorph solid.
IR (KBr, $cm^{-1}$) : 1697, 1481, 1436, 1340, 1169, 1074, 853.
$^1H$ NMR ($CDCl_3$, δ): 2,4 (s, 3H), 3,2 (dd, J=6,6 and 18,3Hz, 1H), 3,6 (dd, J=12,8 and 18,3Hz, 1 H), 5,8 (dd, J=6,6 and 12,8Hz, 1H), 7 (d, J=8,2Hz, 2H), 7,2 (s, 1 H), 7,3-7,4 (m, 6H), 8 (d, J=8,1Hz, 2H), 9 (s, 1 H).

**Example 7:**

N-oxide of N-piperidinyl-5-(4-chlorophenyl)-1-(2,4-dichlorophenyl)-4,5-dihydropyrazole-3-carboxamide

Under nitrogen gas as an inert atmosphere N-piperidinyl-5-(4-chlorophenyl)-1-(2,4-dichlorophenyl)-4,5-dihydropyrazole-3-carboxamide (0,15 g, 332 mmoles) was dissolved in 7 ml of dichloromethane. The resulting solution was ice-cooled to 0 °C and m-chloroperbenzoic acid (0,204 g, 0,83 mmoles) added in several portions.
After stirring for 15 minutes a control via thin layer chromatography showed that no starting material was remaining. A saturated solution of sodium bicarbonate was then slowly added, the organic phase separated, washed with water, dried over sodium sulfate and filtered. The filtered solution was evaporated to dryness and the crude product was purified via column chromatography yielding 78 mg (50 % of theoretical yield) of the N-oxide of N-piperidinyl-5-(4-chlorophenyl)-1-(2,4-dichlorophenyl)-4,5-dihydropyrazole-3-carboxamide in form of a white solid having a melting point of 115-120 °C.
IR (KBr, $cm^{-1}$): 3202, 1678, 1654, 1474, 1309, 1107.
$^1H$-NMR ($CDCl_3$, δ): 1.6 (m, 2H), 1.8-2.0 (m, 4H), 2.55 (m, 2H), 3.3 (dd, J = 6.3 Hz and 18.2 Hz, 1H), 3.7 (m, 3H), 5.8 (dd, J = 6.3 Hz and 12.5 Hz, 1H), 7.0-7.3 (m, 7H), 8.5 (s, 1 H.)

**Example 8:**

**5-(4-chlorophenyl)-1-(2,4-dichlorophenyl)-4,5-dihydro-pyrazole-3-carboxylic acid**

**a) 4-(4-chlorophenyl)-2-oxo-3-butenoic acid**

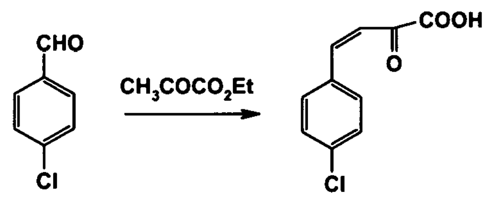


In a three neck flask p-chlorobenzaldehyde (13,3 g, 95 mmoles) and ethyl pyruvate (10 g, 86 mmoles) were dissolved in 150 ml of absolute ethanol. The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$) : 3500-2500, 1719,3, 1686,5, 1603,4, 1587,8, 1081,9.

$^1H$ NMR($CDCl_3$, δ) : 7,4 (d, J=8,4Hz, 2H), 7,5 (d, J=16,1Hz, 1H), 7,6 (d, J=8,4Hz, 2H), 8,1(d, J=16,1Hz, 1H).

**a2) 5-(4-chlorophenyl)-1-(2,4-dichlorophenyl)-4,5-dihydro-pyrazole-3-carboxylic acid**

In an alternative route instead of using ethylpyruvate the salt $CH_3$-C(O)-C(O)-$O^-$ $Na^+$ (sodiumpyruvate) was used, dissolved ethanolic water.

**b) 5-(4-chlorophenyl)-1-(2,4-dichlorophenyl)-4,5-dihydro-pyrazole-3-carboxylic acid**

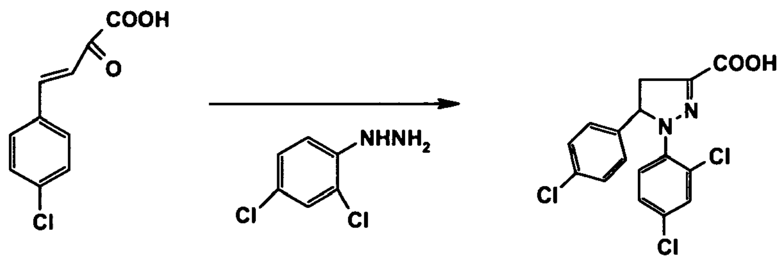

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, $cm^{-1}$) : 3200-2200, 1668,4, 1458, 1251,4, 1104,8.

$^1H$ NMR ($CDCl_3$, δ) : 3,3 (dd, 1H), 3,7 (dd, 1 H), 5,9 (dd, 1H), 7,09-7,25 (m, 7H).

**Example 9:**

**N-piperidinyl-5-(4-chlorophenyl)-1-(2,4-dichlorophenyl)-4,5-dihydro-1H-pyrazole-3-carboxamide**

**(a) 5-(4-chlorophenyl)-1-(2,4-dichlorophenyl)-4,5-dihydro-pyrazole-3-carboxylic acid chloride**

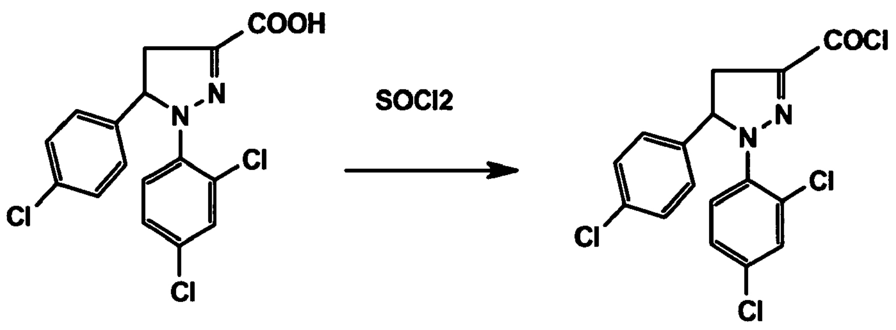


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to Example 0 was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, $cm^{-1}$) : 1732,3, 1700, 1533,3, 1478,1, 1212,9, 826,6.

Starting from this compound compounds according to general formulas II and III wherein $R^1$ is a linear or branched $C_{1-4}$-alkyl group can be prepared reacting this compound with the appropriate alkyl alcohol.

**(b) N-piperidinyl-5-(4-chlorophenyl)-1-(2,4-dichlorophenyl)-4,5-dihydropyrazole-3-carboxamide**

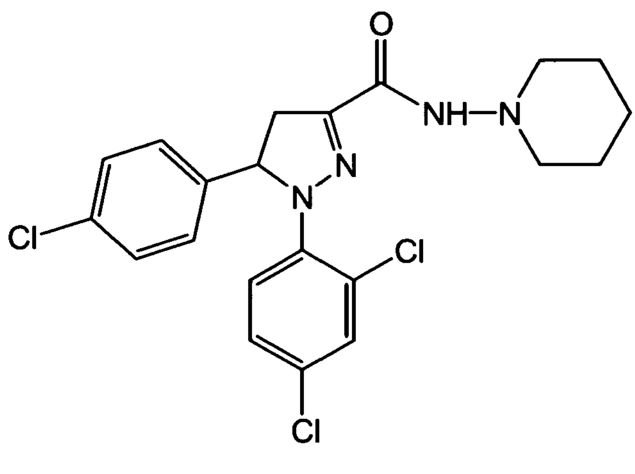

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydro-pyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, $cm^{-1}$): 3222,9, 2934,9, 1647,4, 1474,7, 1268,3, 815,6.

$^1H$ NMR ($CDCl_3$, δ): 1,4 (m, 2H), 1,7 (m, 4H), 2,8 (m, 4H), 3,3 (dd, J=6,1 y 18,3Hz, 1H), 3,7 (dd, J=12,5 and 18,3 Hz, 1H), 5,7 (dd, J=6,1 and 12,5 Hz, 1H), 7,0-7,2 (m, 6H), 7,4 (s, 1H).

The compound according to the following example 9 has been prepared analogously to the process described in Example 1 in combination with Example 0.

**Example 10:**

**5-(4-Chloro-phenyl)-1-(2,4-dichloro-phenyl)-4,5-dihydro-1H-pyrazole-3-carboxylic acid-[1,2,4]triazol-4-yl amide**

Melting point: 134-138 °C.

IR (KBr, $cm^{-1}$): 3448, 1686, 1477, 1243, 1091, 821.

$^1H$ NMR($CDCl_3$, δ): 3,1 (dd, J=6,2 and 17,9Hz, 1 H), 3,7 (dd, J=12,3 and 17,9Hz, 1 H), 5,9 (dd, J=6,2 and 12,3 Hz, 1 H), 7,2-7,5 (m, 7H), 8,7 (s, 2H), 12,0 (bs, 1H).

**Pharmacological Data:**

**I.In-vitro determination of affinity to $CB_1$/$CB_2$-Rezeptors**

The affinity of the inventive substituted pyrazoline compounds to $CB_1$/$CB_2$ receptors was determined as described above. Some of the values obtained are given in the following table I:

Table I:

| Compound according to Example | $CB_1$-Receptor Radiologand:[$^3H$]-CP55940 | | $CB_2$-Receptor Radiologand:[$^3H$]-CP55940 | |
|---|---|---|---|---|
| | % Inhibition $10^{-6}$M | $K_i$(nM) | % Inhibition $10^{-6}$M | $K_i$(nM) |
| 1 | 93% | <25 | 33% | > 1000 |
| 5 | 79% | 111 | 54% | ≈ 1000 |

As can be seen from the values given in table 1 the inventive pyrazoline compounds are particularly suitable for regulating the $CB_1$-Receptor.

**II. In-vivo bioassay system for determination of cannabinoid activity**

The determinination of cannabinoid activity in-vivo was determined as described above. Some of the values obtained are given in the following table II:

Table II:

| Compound according to example: | dosis administered: 5 mg/kg i.v. Agonistic effect | | | | dosis administered 5 mg/kg i.v. prior to Win 55212-2 in a dose of 1,25mg/kg i.v. Antagonistic Effect | | | |
|---|---|---|---|---|---|---|---|---|
| | A | B | C. | D | A | B. | C | D |
| 1 | 0 | 0 | 0 | 0 | 74 | 100 | 100 | 100 |

(continued)

| Compound according to example: | dosis administered: 5 mg/kg i.v. Agonistic effect | | | | dosis administered 5 mg/kg i.v. prior to Win 55212-2 in a dose of 1,25mg/kg i.v. Antagonistic Effect | | | |
|---|---|---|---|---|---|---|---|---|
| | A | B | C. | D | A | B. | C | D |
| 5 | 0 | 50 | 0 | 0 | 50 | 40 | 20 | 20 |

i.v. intravenous
A: Hot-Plate test
B: Hypothermia
C: Catalepsy
D: Sedation

As can be seen from the values given in table II the inventive pyrazoline compounds show an antagonistic effect.

**III. In-vivo testing for antiobesic activity**

The in-vivo testing for antiobesic activity was carried out as described above, whereby four different groups of 10 rats each were treated as follows:

Group I:

Group was treated with vehicle, namely arabic gum (5 wt.-%) in water.

Group II:

The second group of rats was treated with the inventive compound N-piperidinyl-5-(4-chlorophenyl)-1-(2,4-dichlorophenyl)-4,5-dihydropyrazole-3-carboxamide according to Example 1. Said compound was administered intraperitoneally to the rats over a period of 14 days in a daily dosis of (10 mg/kg body weight).

Group III:

The third group of rats was treated with Amphetamine, an active ingredient known to reduce appetite. Said compound was administered intraperitoneally to the rats over a period of 14 days in a daily dosis of (5 mg/kg body weight).
As can be seen from Figure 1 the body weight is lowered due to the administration of the inventive compound according to example 1 and this effect is also observed after the treatment is ended.
Figure 2 shows the reduction of food intake due to the administration of the inventive compound according to example 1.

**IV. In vivo testing for antidepressant activity**

The in-vivo testing for antidepressant activity of the inventive pyrazoline compounds in the water despair test was carried out as described above. In particular, the compound according to example 1 displayed positive effects with respect to immobility time and struggling time.

**V. In-vivo testing for regulation of triglycerides in blood plasma**

The results obtained were the following :

| Group | Diet | Treatment | Triglicerides, whole blood levels (mg/dl) | Relative levels |
|---|---|---|---|---|
| I | Standard | - | 61 | 100% |
| II | High Fat | Vehicle 10 ml/kg/day po | 122.4 (*) | 200.6% |
| III | High Fat | | 67.5 (N.S.) | 110.6% |

(continued)

| Group | Diet | Treatment | Triglicerides, whole blood levels (mg/dl) | Relative levels |
|---|---|---|---|---|
| | | Example 0<br>30 mg/kg/day<br>po | | |
| (*) : p<0.05, Anova followed Bonferroni t-test, compared with Group I.<br>NS : Not significant diference, compared with Group I. | | | | |

The results showed that Group II mice receiving high fat diet had significantly higher triglicerides blood levels than the control Group I. But the administration of the compound according to Example 0 (Group III) improved the triglicerides blood levels, which were not different of the levels of the group I, which received standard diet.

Figure 1 shows the clear reduction of triglyceride levels in blood plasma. The level (Group III) returns to the control level of Group I compared to the clearly raised levels found in the Group II without the treatment with the compound according to Example 0.

Thus, it has been proved the inhibitory effect of the inventive compound 5-(4-chlorophenyl)-1-(2,4-dichlorophenyl)-4,5-dihydro-pyrazole-3-carboxylic acid according to Example 0. on the high blood levels of triglicerides.

References

1.-Lambert P.D. et al.."Cyliary neurotrophic factor activates leptin-like pathways and reduces body fat" P.N.A.S. 2001, 28 (8): 4652-4657

2.- Grasa M.M. et al "Oleoyl-Estrone lowens the body weight of both ob/ob and db/db mice. Hozm. Metab. Res 2000, 32 : 246-250

**VI. In-vivo testing for regulation of triglycerides in blood plasma**

TG (triglyceride) levels were 1.28 ± 25 mmoles/l in the group treated with vehicle and only 0.80 ± 0.07 mmoles/l in the group treated with the inventive compound 5-(4-chlorophenyl)-1-(2,4-dichlorophenyl)-4,5-dihydro-pyrazole-3-carboxylic acid according to Example 0. The results were statistically highly significant with an ANOVA factorial, Fisher's post-hoc test of *** p<0.005 vs. vehicle.

Thus, again the inhibitory effect of the inventive compound 5-(4-chlorophenyl)-1-(2,4-dichlorophenyl)-4,5-dihydro-pyrazole-3-carboxylic acid according to Example 0 on the high blood levels of triglicerides was demonstrated.

## Claims

1. Use of a substituted pyrazoline compound of the general formula I,

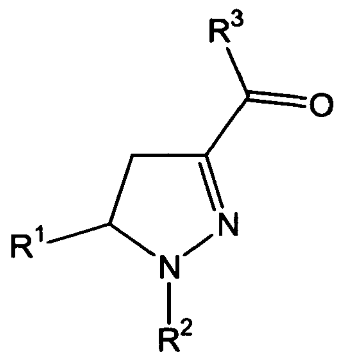

I

wherein

$R^1$ represents an optionally at least mono-substituted phenyl group;
$R^2$ represents an optionally at least mono-substituted phenyl group;
$R^3$ represents a saturated or unsaturated, optionally at least mono-substituted, optionally at least one heteroatom as ring member containing cycloaliphatic group, which may be condensed with an optionally at least mono-substituted mono- or polycyclic ring system; or $R^3$ represents an optionally at least mono-substituted aryl or heteroaryl group, which may be condensed with an optionally at least mono-substituted mono- or polycyclic ring system; or $R^3$ represents an —$NR^4R^5$-moiety,
$R^4$ and $R^5$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group; an —$SO_2$-$R^6$-moiety; or an -$NR^7R^8$-moiety,
$R^6$ represents a linear or branched, saturated or unsaturated, optionally at least mono-substituted aliphatic group; a saturated or unsaturated, optionally at least mono-substituted, optionally at least one heteroatom as ring member containing cycloaliphatic group, which may be condensed with a mono- or polycyclic ring-system; or an optionally at least mono-substituted aryl or heteroaryl group, which may be condensed with a mono-or polycyclic ring system and/or bonded via a linear or branched alkylene group;
$R^7$ and $R^8$, identical or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloaliphatic group, which may be condensed with an optionally at least mono-substituted mono- or polycyclic ring system; or an optionally at least mono-substituted aryl or heteroaryl group, which may be condensed with an optionally at least mono-substituted mono- or polycyclic ring system and/or bonded via a linear or branched alkylene group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof

and/or optionally
a substituted pyrazoline compound of the general formula II,

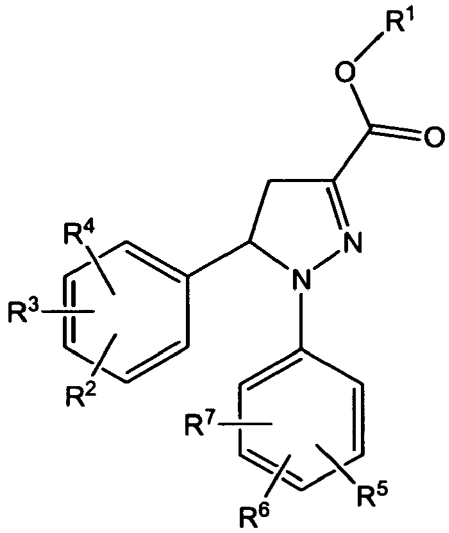

II

wherein

$R^1$ represents hydrogen or a linear or branched $C_{1-4}$-alkyl group,
$R^2$, $R^3$ and $R^4$ independently of each other represent hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^8$, SH, $SR^8$, $SOR^8$, $SO_2R^8$, $NH_2$, $NHR^8$, $NR^8R^9$, -(C=O)-$NH_2$, -(C=O)-$NHR^8$ or -(C=O)-$NR^8R^9$ whereby $R^8$ and $R^9$ for each substituent independently represent linear or branched $C_{1-6}$ alkyl,
$R^5$ and $R^6$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10}$, SH, $SR^{10}$, $SOR^{10}$, $NH_2$, $NHR^{10}$, $NR^{10}R^{11}$, -(C=O)-$NH_2$, -(C=O)-$NHR^{10}$ and -(C=O)-$NR^{10}R^{11}$, whereby $R^{10}$ and optionally $R^{11}$ for each substituent independently represent linear or branched $C_{1-6}$ alkyl;
$R^7$ represents hydrogen, a linear or branched $C_{1-6}$-alkyl group, a linear or branched $C_{1-6}$-alkoxy group, a halogen atom, $CH_2F$, $CHF_2$, $CF_3$, CN, OH, $NO_2$, -(C=O)-$R^{10}$, SH, $SR^{10}$, $SOR^{10}$, $NH_2$, $NHR^{10}$, $NR^{10}R^{11}$, -(C=O)-$NH_2$, -(C=O)-$NHR^{10}$ and -(C=O)-$NR^{10}R^{11}$, whereby $R^{10}$ and optionally $R^{11}$ for each

substituent independently represent linear or branched $C_{1-6}$ alkyl; optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof
and/or optionally a substituted pyrazoline compound of the general formula III

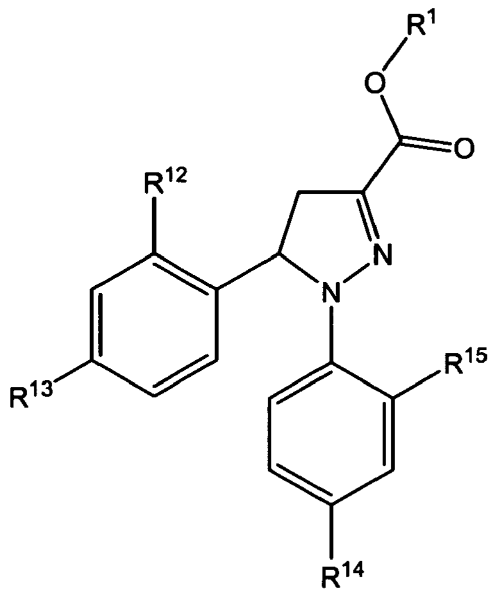

III

wherein

$R^1$ represents hydrogen or a linear or branched $C_{1-4}$-alkyl group,
$R^{12}$ or $R^{13}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, hydrogen, methyl, ethyl, F, Cl, Br and $CF_3$,
$R^{14}$ or $R^{15}$ independently of each other represent a linear or branched $C_{1-6}$-alkyl group, a linear or branched $C_{1-6}$-alkoxy group, a halogen atom, $CH_2F$, $CHF_2$, $CF_3$, CN, OH, $NO_2$, SH, $NH_2$, methyl, ethyl, F, Cl, Br and $CF_3$,

optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof
optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof,

as well as combinations of substituted pyrazoline compounds of the general formula I, II, and/or III one of its/their derivatives, optionally in the form of its/their racemate, pure stereoisomers, especially enantiomers or diastereomers or in the form of mixtures of stereoisomers, especially enantiomers or diastereomers; in any suitable ratio; in neutral form, in the form of an acid or base or in form of a salt, especially a physiologically acceptable salt, or in form of a solvate, especially a hydrate,

for the production of a medicament for the treatment of pain, allodynia, analgesia, angina pain, bone injury pain, cancer pain, central neuropathic pain, central pain, chronic lower back pain, chronic pain, cluster headaches, dental pain, gastrointestinal pain, genitourinary tract-related pain, herpes neuralgia, HIV/AIDS-related pain, inflammatory pain, metabolic neuropathies, neuropathic pain, nociception, nociceptive pain, non-central neuropathic pain, pain associated with de-addiction of drugs, pain associated with spinal cord injury, pain during labor and delivery, pain from cystitis, pain from vascular lesions in the brain, pain resulting from burns, pain resulting from sunburns, pelvic pain, phantom limb pain, post partum pains, post-operative pain, post-stroke pain, Reflex sympathetic dystrophy (RSD), resistant pain, surgical pain, trigeminal pain, visceral pain, amenorrhea, dysmenorrhea, endometriosis, erectile dysfunction, impotence, infertility, menorrhagia, menstrual disorders, premature ejaculation, premature menopause, sexual dysfunction, vaginitis, vulvitis, achalasia, acute or chronic pancreatitis, atrophy of gastric glands, Barrett's metaplasia, carcinoid tumors, chronic erosive gastritis, chronic inflammatory diseases of the bowel, chronic intestinal pseudo-obstruction, colonic inertia, constipation, cricopharyngeal incoordination, Crohn's disease, cystadenocarcinoma, diffuse esophageal spasm, ductal adenocarcinoma, duodenal ulcers, dysphagia, emesis, familial polyposis, functional dyspepsia, gastric ulcers, gastrinoma, gastritis, gastroesophageal reflux, globus sensation, glucagonoma, inflammation of the gastric mucosa, insulinoma, irritable bowel syndrome, islet cell tumors, malabsorption syndrome, megacolon, metaplasia of gastric tissues, multiple endocrine neoplasia syndrome, nausea, neoplasms of the stomach, polyps, pre-esophageal dysphagia, steatorrhea, stress gastritis, ulcerative colitis, vigoruos achalasia, Vipoma syndrome, Zollinger-Ellison syndrome, age-associated memory impairment, age-related cognitive decline, attention deficit disorders, attention deficit hyperactivity disorders, brain injuries, catalepsy, cerebral apoplexy, cerebral ischemia, cerebral vascular accidents, corticobasal degeneration, Creutzfeld Jakob dementia, dementia, dementia with Lewy bodies, frontotemporal dementia, HIV dementia, Korsakoff s psychosis, learning disabilities, memory deficits, mild cognitive impairment, motor neuron disease, multiple sclerosis, neuropathy, Parkinsonism linked to chromosome 17, Pick's disease, post-stroke, post-traumatic brain injury, progressive nuclear palsy, schizophrenia with dementia, small-vessel cerebrovascular disease, thalamic degeneration, traumatic brain injury, vascular cognitive impairment, vascular dementia, atherosclerosis, atrial and ventricular arrhythmias, cardiac insufficiency, congestive heart failure, hypertension, hypertensive vascular diseases, ischemic diseases of the heart, myocardial infarction, peripheral vascular diseases, appetence, appetite disorders, metabolic syndrome, diabetes type I, eating disorders assoc. with excessive food intake, Guillain-Barre syndrome, acute or chronic hepatitis, alpha-antitrypsin-deficiency, ascites, benign neoplasms of the liver, Budd-Chiari syndrome, chronic cholestatic liver disease, cirrhosis of the liver, Crigler-Najjar syndrome, drug-induced disorders of the liver, Dubin-Johnson syndrome, fatty liver, Gaucher's syndrome, Gilbert's syndrome, hepatic granulomas, hepatomegaly, inflammatory conditions of the liver due to viruses, bacteria, fungi, protozoa or helminths, intrahepatic cholestasis, jaundice, liver fibrosis, malignant neoplasms of the liver, portal hypertension, portal-systemicencephalopathy, postoperative intrahepatic cholestasis, primary biliary cirrhosis, primary sclerosing cholangitis, Reye's syndrome, Rotor syndrome, steatosis, viral hepatitis, Wilson's syndrome, convulsions, demyelinisation related disorders, medicament-induced movement disorders, seizures, spinal cord injury, septic shock, viral encephalitis, adenocarcinomas, benign neoplasms, brain tumors, cancer of adipous tissue, cancer of blood vessel, cancer of cartilage tissue, cancer of connective tissue, cancer of muscle tissue, cancer of the endocrine glands, cancer of the gastrointestinal tract, cancer of the penis, cancer of the respiratory tract, cancer of the urogenital system, cancers of blood-forming tissue, carcinoma, carcinosarcoma, dysplasias, hyperplasias, larynx cancer, leukemias, lymphomas, metastasis, metastatic tumors, neck cancer, neoplasms, pituitary cancer, rectum cancer, sarcoma, small intestine, spleen cancer, testes cancer, thyroid cancer, tongue cancer, tumors of nerve tissues, uterus cancer, stress, sedation and spinal cord injury.

European Patent Office

# EUROPEAN SEARCH REPORT

Application Number
EP 05 38 4024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 384 477 A (LABORATORIOS DEL DR. ESTEVE, S.A) 28 January 2004 (2004-01-28)<br>* paragraphs [0011], [0012]; claim 1 *<br>see especially compounds 36, 39 and 42<br>----- | 1 | A61K31/4155<br>A61K31/496<br>A61K31/454 |
| X | EP 1 083 171 A (LABORATORIOS DEL DR. ESTEVE, S.A) 14 March 2001 (2001-03-14)<br>* paragraph [0013] *<br>see especially compounds 34-42<br>----- | 1 | |
| E | WO 2005/074920 A (SOLVAY PHARMACEUTICALS B.V; LANGE, JOSEPHUS H.M; KRUSE, CORNELIS G; VA) 18 August 2005 (2005-08-18)<br>* page 13 - page 14 *<br>see compounds 1-4<br>* table 1 *<br>* page 6, line 12 - line 16 *<br>----- | 1 | |

TECHNICAL FIELDS SEARCHED (IPC)

A61K
A61P

The present search report has been drawn up for all claims

3

| Place of search | Date of completion of the search | Examiner |
|---|---|---|
| Munich | 17 November 2005 | Loher, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 38 4024

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2005

| Patent document cited in search report | | Publication date | Patent family member(s) | | | Publication date |
|---|---|---|---|---|---|---|
| EP 1384477 | A | 28-01-2004 | BR | 0208805 | A | 13-07-2004 |
| | | | CA | 2442974 | A1 | 17-10-2002 |
| | | | CN | 1509171 | A | 30-06-2004 |
| | | | WO | 02080909 | A1 | 17-10-2002 |
| | | | ES | 2174757 | A1 | 01-11-2002 |
| | | | HU | 0400918 | A2 | 28-07-2004 |
| | | | JP | 2004525166 | T | 19-08-2004 |
| | | | MA | 27019 | A1 | 20-12-2004 |
| | | | NO | 20034470 | A | 05-12-2003 |
| | | | PL | 365220 | A1 | 27-12-2004 |
| | | | US | 2004034082 | A1 | 19-02-2004 |
| | | | ZA | 200308626 | A | 05-11-2004 |
| EP 1083171 | A | 14-03-2001 | AT | 265437 | T | 15-05-2004 |
| | | | AU | 752001 | B2 | 05-09-2002 |
| | | | AU | 3932999 | A | 20-12-1999 |
| | | | BG | 105005 | A | 31-08-2001 |
| | | | BR | 9910801 | A | 27-11-2001 |
| | | | CA | 2333475 | A1 | 09-12-1999 |
| | | | CN | 1307566 | A | 08-08-2001 |
| | | | DE | 69916828 | D1 | 03-06-2004 |
| | | | DE | 69916828 | T2 | 14-04-2005 |
| | | | DK | 1083171 | T3 | 09-08-2004 |
| | | | ES | 2137138 | A1 | 01-12-1999 |
| | | | WO | 9962884 | A1 | 09-12-1999 |
| | | | ES | 2221382 | T3 | 16-12-2004 |
| | | | HU | 0102102 | A2 | 28-03-2002 |
| | | | JP | 2002516908 | T | 11-06-2002 |
| | | | LT | 2000108 | A | 25-09-2001 |
| | | | LV | 12632 | A | 20-03-2001 |
| | | | LV | 12632 | B | 20-07-2001 |
| | | | NO | 20006029 | A | 26-01-2001 |
| | | | NZ | 508990 | A | 20-12-2002 |
| | | | PL | 344412 | A1 | 05-11-2001 |
| | | | PT | 1083171 | T | 30-09-2004 |
| | | | RU | 2233272 | C2 | 27-07-2004 |
| | | | SI | 20580 | A | 31-12-2001 |
| | | | SK | 18072000 | A3 | 10-07-2001 |
| | | | TW | 572898 | B | 21-01-2004 |
| | | | UA | 61137 | C2 | 15-03-2001 |
| | | | US | 6353117 | B1 | 05-03-2002 |
| | | | ZA | 200007638 | A | 13-11-2001 |
| WO 2005074920 | A | 18-08-2005 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 20 January 1986
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992, vol. 459
- *Synthetic communications,* 1996, vol. 26 (11), 2229-33
- *Synlett,* 2001, 147-149
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- **KROGSGAARD-LARSEN et al.** Textbook of Drugdesign and Discovery. Taylor & Francis, April 2002
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** International Union of Pharmacology XXVII. Classification of Cannabinoid Receptors. *Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol.,* 2003, vol. 138 (4), 544-553
- **LAMBERT P.D. et al.** Cyliary neurotrophic factor activates leptin-like pathways and reduces body fat. *P.N.A.S.,* 2001, vol. 28 (8), 4652-4657
- **GRASA M.M. et al.** Oleoyl-Estrone lowens the body weight of both ob/ob and db/db mice. *Hozm. Metab. Res,* 2000, vol. 32, 246-250